# EUROPEAN PATENT APPLICATION

(11) **EP 1 878 792 A1**
(43) Date of publication of application: **16.01.2008**
(21) Application number: 06014509.1
(22) Date of filing: 12.07.2006
(51) Int. Cl.: C12N 9/88, C12N 15/00

(54) **Polynucleotides encoding caryophyllene synthase and uses thereof**

(71) Applicant: MAX-PLANCK-GESELLSCHAFT ZUR FÖRDERUNG DER WISSENSCHAFTEN E.V., 80539 München (DE)
(72) Inventor: Degenhardt, Jörg, 06618 Naumburg (DE); Gershenzon, Jonathan, 07745 Jena (DE); Köllner, Tobias G., 07768 Grosseutersdorf (DE)
(74) Representative: Vossius & Partner

(57) **Abstract**

Described are polynucleotides encoding a caryophyllene synthase. Also described are recombinant nucleic acid molecules and vectors comprising said polynucleotide as well as host cells genetically engineered with said polynucleotides. Further described are caryophyllene synthase polypeptides encoded by said polynucleotide as well as binding molecules specifically recognizing said polypeptide. Additionally, described are transgenic plant cells, plant tissues and plants genetically engineered with the caryophyllene synthase-encoding polynucleotide, corresponding methods for the production of said transgenic plant cells, plant tissues and plants and corresponding uses and methods using said polynucleotide for establishing or enhancing resistance against root-damaging insects in plants, particularly resistance against the corn rootworm in maize plants. In addition, described are regulatory sequences derived from the caryophyllene synthase-encoding gene described herein and corresponding compounds, methods and uses relating to applications of said regulatory sequence. Moreover, described is a method for breeding a plant having a resistance against a root-damaging insect comprising a selection step for progenitor lines and/or offspring that express said caryophyllene synthase polypeptide.

## Description

The present invention relates to polynucleotides encoding a caryophyllene synthase. The present invention furthermore relates to recombinant nucleic acid molecules and vectors comprising said polynucleotide as well as to host cells genetically engineered with said polynucleotides. The present invention also relates to caryophyllene synthase polypeptides encoded by said polynucleotides as well as to binding molecules specifically recognizing said polypeptide. Furthermore, the present invention relates to transgenic plant cells, plant tissues and plants genetically engineered with the caryophyllene synthase-encoding polynucleotide, to corresponding methods for the production of said transgenic plant cells, plant tissues and plants and to corresponding uses and methods using said polynucleotide for establishing or enhancing resistance against root-damaging insects in plants, particularly resistance against the corn rootworm in maize plants. In addition, the present invention relates to a regulatory sequence derived from the caryophyllene synthase-encoding gene described herein and to corresponding compounds, methods and uses relating to applications of said regulatory sequence. Moreover, the present invention relates to a method for breeding a plant having a resistance against a root-damaging insect comprising a selection for progenitor lines and/or offspring that express said caryophyllene synthase polypeptide.

Environmentally desirable protection of crops against arthropod herbivores can inter alia use biological control, which depends on the use of antagonists or enemies of the pest organisms. To be effective, it is crucial that the biological control agents are able to find their prey efficiently enough to minimize damage to the crop.

More than a decade ago it was discovered that when herbivores feed, the plants produce volatiles that are attractive to the natural enemies of the herbivore (Dicke, Proceedings of Semiochemicals and Pest Control - Prospects for New Applications, 1990, Wageningen, the Netherlands, pp. 3091-3118; and Turlings, Science 250 (1990), 1251-1853). Thus, plants indirectly defend themselves by enhancing the effectiveness of the natural enemies of the herbivores. The use of predators and parasitoids for biological control is receiving more and more attention and for many years it has been common practice in a number of crops in glasshouse as well as open fields (Van Lenteren, In: Biological control: Measures of Success, eds. G. Gurr & S. Wratten, 2000, Kluwer Academic Publishers, Dordrecht; Kfir, Annual Rev. Entomol. 47 (2002), 701-731). Nevertheless, breeders and agronomists have so far paid little attention to the optimisation of biological control. This is probably due to the relatively recent discovery of the phenomenon of indirect defence, the more complex relationships involved and the difficulties associated with quantification of the effects.

The beetle *Diabrotica virgifera* (a species of the corn rootworm) is an agronomically important maize pest in North America that attacks the roots of the plants. It has recently been introduced into Europe and is now quickly spreading within Germany and other European countries. Since the larvae of these beetles live in the soil and damage the roots of the maize plant, it has been very difficult and expensive to control this pest through pesticide applications.

In the work of Rasmann et al. (Nature 434 (2005), 732-737), it was recently demonstrated that some maize varieties are capable to defend themselves against Diabrotica utilizing an indirect defence mechanism. After damage by Diabrotica larvae, the roots of these plants release beta-caryophyllene, a sesquiterpene hydrocarbon, into the soil. This compound attracts nematodes from the surrounding soil that feed on the Diabrotica larvae, eventually killing them. Thereby, the release of caryophyllene leads to an overall benefit to the plants in laboratory experiments. The first field experiments also suggest a reduced Diabrotica damage due to caryophyllene emission.

A similar system is known from the aerial parts of maize plants which, when attacked by caterpillars, release a mixture of odorous compounds that attract parasitic wasps, natural enemies of the herbivores. In a study reported by Degen et al. (Plant Physiol. 135 (2004), 1928-1938), European and American maize inbred lines were examined for their capacity to release these odorous compounds. Thereby, it was found out that European maize varieties are mostly able to produce caryophyllene while almost all American varieties have lost this ability. It is therefore assumed that American varieties are less well defended against Diabrotica due to their inability to produce the caryophyllene signal (Rasmann, loc. cit.). This assumption is currently being tested both in laboratory and field trials.

In view of this, it would be desirable to elucidate the molecular basis of the caryophyllene production in maize plants in order to be able to improve resistance against Diabrotica pest, particularly in North American varieties, more directly.

Thus, the technical problem underlying the present invention is to provide means and methods for enhancing caryophyllene production in the roots of plants in order to increase the plant's resistance against root-damaging insects.

This technical problem is solved by the provision of the embodiments as characterized in the claims.

Accordingly, the present invention relates to polynucleotides selected from the group consisting of
(a) polynucleotides comprising a nucleotide sequence encoding a polypeptide having the amino acid sequence of SEQ ID NO:2 or 4;
(b) polynucleotides comprising the nucleotide sequence shown in SEQ ID NO:1 or 3;
(c) polynucleotides comprising a nucleotide sequence encoding a fragment of the polypeptide encoded by a polynucleotide of (a) or (b), wherein said nucleotide sequence encodes a protein having caryophyllene synthase activity;
(d) polynucleotides comprising a nucleotide sequence the complementary strand of which hybridizes to the polynucleotide of any one of (a) to (c), wherein said nucleotide sequence encodes a protein having caryophyllene synthase activity; and
(e) polynucleotides comprising a nucleotide sequence that deviates from the nucleotide sequence defined in (d) by the degeneracy of the genetic code.

The present invention is based on the isolation of the gene TPS23 encoding a polypeptide having caryophyllene synthase activity from the maize variety Delprim. This maize variety was shown to synthesize (E)-β-caryophyllene, a sesquiterpene which attracts the entomopathogenic nematode Heterorhabditis megidis which is a predator of the root pest Diabrotica virgifera (Rasmann, loc. cit.).
Thus, it is contemplated that the polynucleotide of the present invention may be used for providing plants that have an increased resistance against root-damaging insects, either by producing corresponding transgenic plants or by applying molecular marker-assisted non-transgenic breeding methods.
In connection with the present invention, the term "resistant" or "resistance" refers to the property of a given plant or plant species to protect itself against an attack by a certain root-damaging insect, whereby said protection may range from a significant reduction to a complete inhibition of herbivory. The type of resistance envisaged in connection with the present invention is based on a so-called tritrophic interaction between a plant, a root-damaging insect and an entomopathogenic nematode (cf. Bouwmeester, Proceedings of BCPC International Congress Crop Science & Technology 2003, 10-12 November 2003, Glasgow, Scotland, Vol 2, 1123-1134). In such a system, the feeding of a herbivore (e.g. a root-damaging insect) leads to the release of one or more volatile compounds (e.g. caryophyllene) which attract one or more predators specialized for the herbivore (e.g. entomopathogenic nematodes). This is described in Rasmann (loc. cit.) for a tritrophic interaction between maize plants, the corn rootworm and the nematode Heterorhabditis megidis. As is apparent from the tritrophic plant defense system on which the present invention is based, the resistance conferred to a plant by expressing the polynucleotide of the invention presupposes that at least one suitable entomopathogenic nematode that is attracted by caryophyllene and that attacks the root-damaging insect is available in the soil surrounding the plant. In order to fulfill this requirement, suitable nematodes may be naturally present in the soil and/or may be added as a biological pest control agent. The nematodes may be added to the soil together with a nutrient suitable for them. As is typical for biological pest control systems, the use of a biological enemy of the pest organism might not lead to the complete elimination of the pest, but will reduce the levels of the pest organism below those resulting in agronomic significant damage. The resulting levels of the pest organism will be dependent on the populations of entomopathogenic nematodes that are either naturally occurring in the soil or will be added to the soil. The already established agronomic procedures of insecticide treatment to reduce the concentration of the adult Diabrotica beetle, soil insecticide treatments to reduce the concentration of the Diabrotica larvae and insecticide treatment of the seeds can be combined with the herein described biological defense strategies.

Successfully established resistance in a plant according to the present invention may be measured by assays described in the prior art, in particular in Rasmann (loc. cit.). Accordingly, a plant produced according to the present invention may be exposed to a root-damaging insect and, subsequently, its capacity to attract entomopathogenic nematodes as compared to a corresponding control plant may be examined. This may be done by using a six-arm olfactometer as disclosed in Rasmann (loc. cit.). In such an assay, a plant according to the present invention shows an attraction of entomopathogenic nematodes which is significantly higher than in the control plant, preferably at least 20%, more preferably at least 50% and most preferably at least 100% higher.

Alternatively, resistance can be detected by measuring the number of herbivores (larvae) around one plant, the number of nematode-infected herbivores around one plant or the number of adult insects emerging around one plant, in comparison to a corresponding control plant. Corresponding assays which were carried out in the field are also described in Rasmann (loc. cit.). Accordingly, with respect to plants of the present invention, the number of herbivores (larvae) around one plant is significantly reduced, preferably by at least 10%, more preferably by at least 20%, as compared to a control plant. Furthermore, the amount of nematode-infected herbivores (larvae) is increased in plants according to the present invention significantly, preferably by at least 100%, more preferably by at least 200% and even more preferably by at least 300% as compared to the control plant. The number of adult insects emerging around one plant is significantly decreased for plants of the present invention as compared to the control plant, preferably by at least 10%, more preferably by at least 20% and even more preferably by at least 30%.

The term "root-damaging insect" as used herein refers to insect larvae which feed on plant roots. In the framework of the present invention, this term particularly refers to larvae of the genus Diabrotica (interchangeably also referred to herein as "corn rootworm") and, especially, to larvae of the species Diabrotica virgifera, and more preferably to larvae of the subspecies Diabrotica virgifera virgifera (Western corn rootworm, WCR).
The term "caryophyllene synthase activity" in connection with the present invention refers to the activity of a polypeptide encoded by the polynucleotide of the invention to catalyze the conversion of (E,E)-farnesyl diphosphate (FPP) to (E)-β-caryophyllene.

The activity can be tested for by assays for caryophyllene synthase activity known in the prior art, such as in Cai, Phytochemistry 61 (2002), 523-529, or by assays as described in Examples 2 and 3 of the present application.

Preferably, the caryophyllene synthase of the invention does not accept geranyl diphosphate (GPP) or geranyl-geranyl diphosphate (GGPP) as substrate. Furthermore, the caryophyllene synthase of the invention preferably produces α-humulene and/or δ-elemene as minor side products. It is furthermore preferred that the caryophyllene synthase of the invention has the following biochemical properties:
(i) a catalytic optimum from pH 8.0 to pH 9.5;
(ii) a Kₘ value for FPP of 2.4 ± 0.4 µM;
(iii) a k_{cat} value of 0.0030 ± 0.0002 s⁻¹; and/or
(iv) a requirement for a divalent metal ion cofactor, preferably Mg²⁺ ions at 10 mM (particularly with a Kₘ of 183 ± 34 µM) or Mn²⁺ ions at 0.25 mM (particularly with a Kₘ of 28 ± 6 µM).

As a further preferred characteristic, the polynucleotide of the invention, in particular the caryophyllene synthase gene in its natural source organism, shows inducible expression upon attack of the plant root by a root-damaging insect.

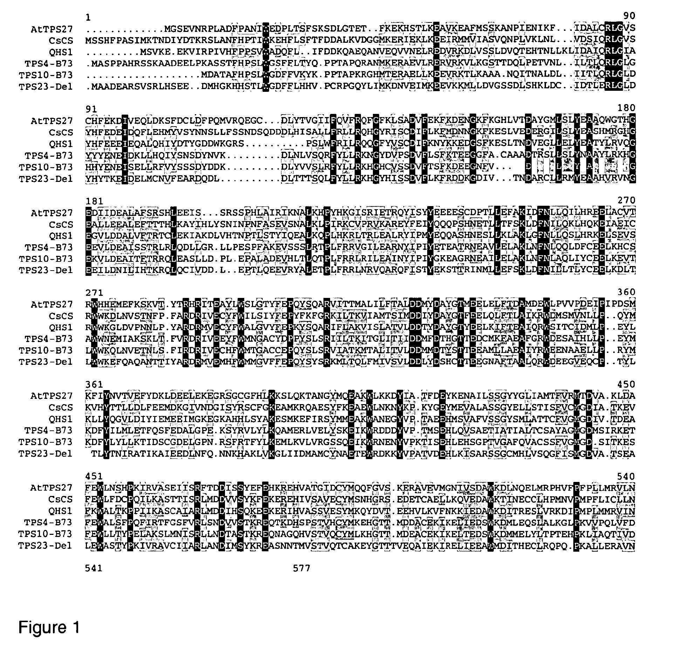

It is furthermore preferred that the expression of the polynucleotide of the invention is not induced upon mechanical lesion of the root or at least only to a significantly smaller degree than in the case of insect infestation.
The polynucleotide of the invention preferably comprises the 1.644 bp coding sequence shown in SEQ ID NO: 1 and, particularly preferably, encodes a protein with a predicted molecular mass of 63.6 kDa. As is evident from Figure 1, numerous amino acids throughout the coding sequence are highly conserved among members of the terpene synthase (TPS) family. The most characteristic element is an Asp-rich DDxxD motif in the C-terminal part of the protein that is involved in the binding of the divalent metal cofactor (Starks et al., 1997). The gene has only a very low amino acid identity to other maize terpene synthases like TPS10 (40.5%, Schnee et al., 2006) and TPS4 (37.8%, Köllner et al., 2004). In the plant material under investigation, no genes with higher sequence identity were found after repeated PCR with maize cDNA and RACE libraries as well as in the maize genomic databases, suggesting that TPS23 is a single gene.

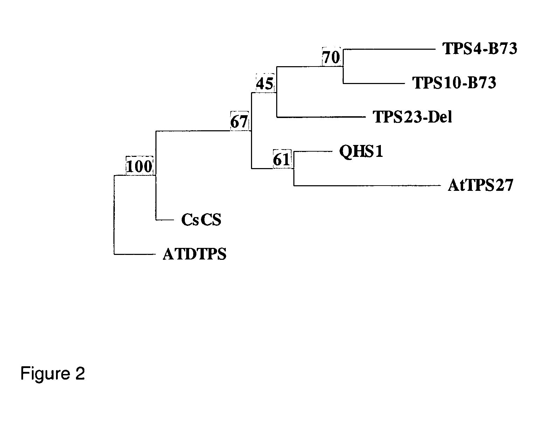

Moreover, TPS23 is the first caryophyllene synthase identified in a monocotyledonous plant. It exhibits a low amino acid identity to caryophyllene synthases known from dicotyledonous plants, i.e. 32.9% with AtTPS27 from Arabidopsis (Aubourg, Mol. Genet. Genomics 267 (2002), 730-745; Chen, Plant Cell 15 (2003), 481-494), 30.3% with CsCS from Cucumis sativus (Mercke, Plant Physiol. 135 (2004), 2012-2024) and 35.1% with QHS1 from Artemisia annua (Cai, Phytochemistry 61 (2002), 523-529). Surprisingly, a dendrogram analysis demonstrates that TPS23 is more closely related to functionally unrelated terpene synthases of maize than to terpene synthases of similar function in other plant species, suggesting a convergent evolution of TPS23 (Figure 2).
Parts of the coding sequence depicted in SEQ ID NO: 1 were already disclosed in the public maize genome database (http://maize.tigr.org/) in the entries AZM4_53695 and AZM4_20519 (the sequences being depicted herein under SEQ ID NOs:7 and 8, respectively). In addition, in the database Plant GDB, the sequence of a maize genomic fragment was disclosed (AC147506 clone ZMMBBc0299P16) which covers the entire coding sequence (SEQ ID NO: 9). However, it is unlikely that this genomic sequence encodes a functional caryophyllene synthase since the exonic sequences show some errors (nucleotide exchanges and frame-shifts with respect to the nucleotide sequence of SEQ ID NO: 1 and an intron-exon junction not being correct). The nucleotide sequences of SEQ ID NOs: 7 to 9, as well as parts thereof as far as they correspond with the above-described TPS23 coding sequence or functional fragments thereof are excluded from the scope of the present invention.
Up to now, it is not possible to predict, solely based on the amino acid sequence of a terpene synthase, which exact reaction the enzyme catalyzes. Thus, the activity of TPS23 could only be determined by recombinantly expressing the enzyme and assaying the activity in a biochemical test system (Examples 2 and 3).

As indicated above, in addition to polynucleotides comprising a nucleotide sequence which encodes the amino acid sequence of SEQ ID NO: 2 or 4 or comprising the nucleotide sequence of SEQ ID NO: 1 or 3, the present invention also refers to fragments of these polynucleotides as well as to polynucleotides comprising a nucleotide sequence the complementary strand of which hybridizes to these polynucleotides, wherein said fragments and hybridizing polynucleotides encode a protein having caryophyllene synthase activity.
The present invention also relates to polynucleotides which encode a polypeptide, which has a homology, that is to say a sequence identity, of at least 30%, preferably of at least 40%, more preferably of at least 50%, even more preferably of at least 60% and particularly preferred of at least 70%, especially preferred of at least 80% and even more preferred of at least 90% to the entire amino acid sequence as indicated in SEQ ID NO: 2 or 4, the polypeptide having caryophyllene synthase activity.
Moreover, the present invention relates to polynucleotides which encode a polypeptide having caryophyllene synthase activity and the nucleotide sequence of which has a homology, that is to say a sequence identity, of at least 40%, preferably of at least 50%, more preferably of at least 60%, even more preferably of more than 65%, in particular of at least 70%, especially preferred of at least 80%, in particular of at least 90% and even more preferred of at least 95°/o when compared to the coding sequence shown in SEQ ID NO: 1 or 3.
It is particular!y preferred that polynucleotides of the invention that encode a polypeptide having caryophyllene synthase activity show the structural characteristics described above for TPS23.
The present invention also relates to polynucleotides, which encode a polypeptide having caryophyllene synthase activity and the sequence of which deviates from the nucleotide sequences of the above-described polynucleotides due to the degeneracy of the genetic code.
The invention also relates to polynucleotides comprising a nucleotide sequence which is complementary to the whole or a part of one of the above-mentioned sequences.
The polynucleotide of the invention is understood to be isolated. The term "isolated" means that the polynucleotide addressed herein is not in the form as it occurs naturally, if it indeed has a naturally occurring counterpart. Accordingly, the other compounds of the invention described further below are understood to be isolated.

In the context of the present invention the term "hybridization" means hybridization under conventional hybridization conditions, preferably under stringent conditions, as for instance described in Sambrook and Russell (2001), Molecular Cloning: A Laboratory Manual, CSH Press, Cold Spring Harbor, NY, USA. In an especially preferred embodiment, the term "hybridization" means that hybridization occurs under the following conditions:
- Hybridization buffer:: 2 x SSC; 10 x Denhardt solution (Fikoll 400 + PEG + BSA; ratio 1:1:1); 0.1% SDS; 5 mM EDTA; 50 mM Na₂HPO₄;
250 µg/ml of herring sperm DNA; 50 µg/ml of tRNA;
or
0.25 M of sodium phosphate buffer, pH 7.2;
1 mM EDTA
7% SDS
- Hybridization temperature T: =60°C
- Washing buffer:: 2 x SSC; 0.1 % SDS
- Washing temperature T: = 60°C.

Polynucleotides which hybridize with the polynucleotides of the invention can, in principle, encode a polypeptide having caryophyllene synthase activity from any organism expressing such polypeptides or can encode modified versions thereof.

Polynucleotides which hybridize with the polynucleotides disclosed in connection with the invention can for instance be isolated from genomic libraries or cDNA libraries of bacteria, fungi, plants or animals. Preferably, such polynucleotides are from plant origin, particularly preferred from a plant belonging to the monocotyledons, more preferably from the family of Poaceae and even more preferably from the genus Zea. Preferably, the polynucleotide of the invention is a variant, preferably an ortholog of a polynucleotide comprising SEQ ID NO:1 or 3 and may for example comprise a nucleotide sequence that originates from an agronomically important crop species such as from maize, wheat, barley, oat, rye, rice or sorghum. Alternatively, such polynucleotides can be prepared by genetic engineering or chemical synthesis.
Such hybridizing polynucleotides may be identified and isolated by using the polynucleotides described hereinabove or parts or reverse complements thereof, for instance by hybridization according to standard methods (see for instance Sambrook and Russell (2001), Molecular Cloning: A Laboratory Manual, CSH Press, Cold Spring Harbor, NY, USA). Polynucleotides comprising the same or substantially the same nucleotide sequence as indicated in SEQ ID NO: 1 or 3 or parts thereof can, for instance, be used as hybridization probes. The fragments used as hybridization probes can also be synthetic fragments which are prepared by usual synthesis techniques, and the sequence of which is substantially identical with that of a polynucleotide according to the invention.
The molecules hybridizing with the polynucleotides of the invention also comprise fragments, derivatives and allelic variants of the above-described polynucleotides encoding a polypeptide having caryophyllene synthase activity. Herein, fragments are understood to mean parts of the polynucleotides which are long enough to encode the described polypeptide, preferably showing the biological activity of a polypeptide of the invention as described above. In this context, the term derivative means that the sequences of these molecules differ from the sequences of the above-described polynucleotides in one or more positions and show a high degree of homology to these sequences, preferably within the preferred ranges of homology mentioned above.
Preferably, the degree of homology is determined by comparing the respective sequence with the nucleotide sequence of the coding region of SEQ ID NO: 1 or 3. When the sequences which are compared do not have the same length, the degree of homology preferably either refers to the percentage of nucleotide residues in the shorter sequence which are identical to nucleotide residues in the longer sequence or to the percentage of nucleotides in the longer sequence which are identical to nucleotide sequence in the shorter sequence. The degree of homology can be determined conventionally using known computer programs such as the DNASTAR program with the ClustalW analysis. This program can be obtained from DNASTAR, Inc., 1228 South Park Street, Madison, WI 53715 or from DNASTAR, Ltd., Abacus House, West Ealing, London W13 0AS UK (support@dnastar.com) and is accessible at the server of the EMBL outstation.
When using the Clustal analysis method to determine whether a particular sequence is, for instance, 80% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.
Preferably, the degree of homology of the hybridizing polynucleotide is calculated over the complete length of its coding sequence. It is furthermore preferred that such a hybridizing polynucleotide, and in particular the coding sequence comprised therein, has a length of at least 300 nucleotides, preferably at least 500 nucleotides, more preferably of at least 750 nucleotides, even more preferably of at least 1000 nucleotides, and most preferred of at least 1500 nucleotides.
Preferably, sequences hybridizing to a polynucleotide according to the invention comprise a region of homology of at least 90%, preferably of at least 93%, more preferably of at least 95%, still more preferably of at least 98% and particularly preferred of at least 99% identity to an above-described polynucleotide, wherein this region of homology has a length of at least 500 nucleotides, more preferably of at least 750 nucleotides, even more preferably of at least 1000 nucleotides and most preferred of at least 1500 nucleotides.
Homology, moreover, means that there is a functional and/or structural equivalence between the corresponding polynucleotides or polypeptides encoded thereby. Polynucleotides which are homologous to the above-described molecules and represent derivatives of these molecules are normally variations of these molecules which represent modifications having the same biological function. They may be either naturally occurring variations, for instance sequences from other ecotypes, varieties, species, etc., or mutations, and said mutations may have formed naturally or may have been produced by deliberate mutagenesis. Furthermore, the variations may be synthetically produced sequences. The allelic variants may be naturally occurring variants or synthetically produced variants or variants produced by recombinant DNA techniques. Deviations from the above-described polynucleotides may have been produced, e.g., by deletion, substitution, insertion and/or recombination.
The polypeptides encoded by the different variants of the polynucleotides of the invention possess certain characteristics they have in common. These include for instance biological activity, molecular weight, immunological reactivity, conformation, etc., and physical properties, such as for instance the migration behavior in gel electrophoreses, chromatographic behavior, sedimentation coefficients, solubility, spectroscopic properties, stability, pH optimum, temperature optimum etc.
The biological activity of a polypeptide of the invention, in particular the capacity to synthesize caryophyllene, can be tested in conventional enzyme assays using the substrate of the polypeptide or a suitable modified form thereof.

The polynucleotides of the invention can be DNA molecules, in particular genomic DNA or cDNA. Moreover, the polynucleotides of the invention may be RNA molecules. The polynucleotides of the invention can be obtained for instance from natural sources or may be produced synthetically or by recombinant techniques, such as PCR, and include modified or derivatized nucleic acid molecules as can be obtained by applying techniques described in the pertinent literature.

In a further embodiment, the invention relates to polynucleotides (including oligonucleotides) specifically hybridizing with a polynucleotide of the invention as described above or with the complementary strand thereof. Such polynucleotides have a length of preferably at least 10, in particular at least 15, and particularly preferably of at least 50 nucleotides. Advantageously, their length does not exceed a length of 1000, preferably 500, more preferably 200, still more preferably 100 and most preferably 50 nucleotides. They are characterized in that they specifically hybridize to the polynucleotides of the invention, that is to say that they do not or only to a very minor extent hybridize to nucleotide sequences encoding another terpene synthase or another caryophyllene synthase such as those from dicotyledons described in the prior art.

The polynucleotides of the present embodiment can be used for instance as primers for amplification techniques such as the PCR reaction or as a hybridization probe to isolate related genes. The hybridization conditions and homology values described above in connection with the polynucleotide encoding a polypeptide having caryophyllene synthase activity may likewise apply in connection with the hybridizing polynucleotides mentioned herein.

In another aspect, the present invention relates to recombinant nucleic acid molecules comprising the polynucleotide of the invention described above. The term "recombinant nucleic acid molecule" refers to a nucleic acid molecule which contains in addition to a polynucleotide of the invention as described above at least one further heterologous coding or non-coding nucleotide sequence. The term "heterologous" means that said polynucleotide originates from a different species or from the same species, however, from another location in the genome than said added nucleotide sequence or is synthetic. The term "recombinant" implies that nucleotide sequences are combined into one nucleic acid molecule by the aid of human intervention. The recombinant nucleic acid molecule of the invention can be used alone or as part of a vector.
For instance, the recombinant nucleic acid molecule may encode the polypeptide having caryophyllene synthase activity fused to a marker sequence, such as a peptide, which facilitates purification of the fused polypeptide. The marker sequence may for example be a hexa-histidine peptide, such as the tag provided in a pQE vector (Qiagen, inc.) or the His tag used in Example 2 (see infra), which provide for convenient purification of the fusion polypeptide. Another suitable marker sequence may be the HA tag which corresponds to an epitope derived from influenza hemagglutinin polypeptide (Wilson, Cell 37 (1984), 767). As a further example, the marker sequence may be glutathione-S-transferase (GST) which, apart from providing a purification tag, enhances polypeptide stability, for instance, in bacterial expression systems.

In a preferred embodiment, the recombinant nucleic acid molecules further comprise expression control sequences operably linked to the polynucleotide comprised in the recombinant nucleic acid molecule. More preferably, these recombinant nucleic acid molecules are expression cassettes.
The term "operatively linked" or "operably linked", as used throughout the present description, refers to a linkage between one or more expression control sequences and the coding region in the polynucleotide to be expressed in such a way that expression is achieved under conditions compatible with the expression control sequence.
Expression comprises transcription of the heterologous DNA sequence, preferably into a translatable mRNA. Regulatory elements ensuring expression in prokaryotic as well as in eukaryotic cells, preferably in plant cells, are well known to those skilled in the art. They encompass promoters, enhancers, termination signals, targeting signals and the like. Examples are given further below in connection with explanations concerning vectors. In the case of eukaryotic cells, expression control sequences may comprise poly-A signals ensuring termination of transcription and stabilization of the transcript, for example, those of the 35S RNA from Cauliflower Mosaic Virus (CaMV) or the nopaline synthase gene from Agrobacterium tumefaciens. Additional regulatory elements may include transcriptional as well as translational enhancers. A plant translational enhancer often used is the CaMV omega sequences. Similarly, the inclusion of an intron (e.g. intron-1 from the shrunken gene of maize) has been shown to increase expression levels by up to 100-fold (Mait, Transgenic Research 6 (1997), 143-156; Ni, Plant Journal 7 (1995), 661-676).

Moreover, the invention relates to vectors, in particular plasmids, cosmids, viruses, bacteriophages and other vectors commonly used in genetic engineering, which contain the above-described polynucleotides or recombinant nucleic acid molecules of the invention. In a preferred embodiment, the vectors of the invention are suitable for the transformation of fungal cells, cells of microorganisms such as yeast or bacterial cells, animal cells or, in particular, plant cells. In a particularly preferred embodiment such vectors are suitable for stable transformation of plants.

In another preferred embodiment, the vectors further comprise expression control sequences operably linked to said polynucleotides contained in the vectors. These expression control sequences may be suited to ensure transcription and synthesis of a translatable RNA in prokaryotic or eukaryotic cells.
The expression of the polynucleotides of the invention in prokaryotic or eukaryotic cells, for instance in *Escherichia coli,* is interesting because it permits a more precise characterization of the biological activities of the encoded polypeptide. In particular, it is possible to express these polypeptides in such prokaryotic or eukaryotic cells which are free from interfering polypeptides. In addition, it is possible to insert different mutations into the polynucleotides by methods usual in molecular biology (see for instance Sambrook and Russell (2001), Molecular Cloning: A Laboratory Manual, CSH Press, Cold Spring Harbor, NY, USA), leading to the synthesis of polypeptides possibly having modified biological properties. In this regard it is on the one hand possible to produce deletion mutants in which polynucleotides are produced by progressive deletions from the 5' or 3' end of the coding DNA sequence, and said polynucleotides lead to the synthesis of correspondingly shortened polypeptides.
On the other hand, the introduction of point mutations is also conceivable at positions at which a modification of the amino acid sequence for instance influences the biological activity or the regulation of the polypeptide.
Moreover, mutants possessing a modified substrate or product specificity can be prepared. Furthermore, it is possible to prepare mutants having a modified activity-temperature-profile. Preferably, such mutants show an increased activity. Alternatively, mutants can be prepared the catalytic activity of which is abolished without loosing substrate binding activity.
Furthermore, in the case of expression in plants, plant tissue or plant cells, the introduction of mutations into the polynucleotides of the invention allows the gene expression rate and/or the activity of the polypeptides encoded by the polynucleotides of the invention to be reduced or increased.
For genetic engineering in prokaryotic cells, the polynucleotides of the invention or parts of these molecules can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, in vitro mutagenesis, "primer repair", restriction or ligation can be used. In general, a sequence analysis, restriction analysis and other methods of biochemistry and molecular biology are carried out as analysis methods.

Additionally, the present invention relates to a method for producing genetically engineered host cells comprising introducing the above-described polynucleotides, recombinant nucleic acid molecules or vectors of the invention into a host cell.

Another embodiment of the invention relates to host cells, in particular prokaryotic or eukaryotic cells, genetically engineered with the above-described polynucleotides, recombinant nucleic acid molecules or vectors of the invention or obtainable by the above-mentioned method for producing genetically engineered host cells, and to cells derived from such transformed cells and containing a polynucleotide, recombinant nucleic acid molecule or vector of the invention. In a preferred embodiment the host cell is genetically modified in such a way that it contains the polynucleotide stably integrated into the genome. Preferentially, the host cell of the invention is a bacterial, yeast, fungus, plant or animal cell.
More preferably the polynucleotide can be expressed so as to lead to the production of a polypeptide having caryophyllene synthase activity. An overview of different expression systems is for instance contained in Methods in Enzymology 153 (1987), 385-516, in Bitter et al. (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440). An overview of yeast expression systems is for instance given by Hensing et al. (Antonie van Leuwenhoek 67 (1995), 261-279), Bussineau et al. (Developments in Biological Standardization 83 (1994), 13-19), Gellissen et al. (Antonie van Leuwenhoek 62 (1992), 79-93, Fleer (Current Opinion in Biotechnology 3 (1992), 486-496), Vedvick (Current Opinion in Biotechnology 2 (1991), 742-745) and Buckholz (Bio/Technotogy 9 (1991), 1067-1072).
Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E. coli,* S. *cerevisiae)* are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.
The transformation of the host cell with a polynucleotide or vector according to the invention can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc. The polypeptide according to the present invention can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Polypeptide refolding steps can be used, as necessary, in completing configuration of the polypeptide. Finally, high performance liquid chromatography (HPLC) can be employed for final purification steps.

Accordingly, the present invention also relates to a method for the production of a polypeptide encoded by a polynucleotide of the invention as described above in which the above-mentioned host cell is cultivated under conditions allowing for the expression of the polypeptide and in which the polypeptide is isolated from the cells and/or the culture medium.

Moreover, the invention relates to a polypeptide which is encoded by a polynucleotide according to the invention or obtainable by the above-mentioned method for the production of a polypeptide.
The polypeptide of the present invention may, e.g., be a naturally purified product or a product of chemical synthetic procedures or produced by recombinant techniques from a prokaryotic or eukaryotic host (for example, by bacterial, yeast, higher plant, insect and mammalian cells in culture). Depending upon the host employed in a recombinant production procedure, the polypeptide of the present invention may be glycosylated or may be non-glycosylated. The polypeptide of the invention may also include an initial methionine amino acid residue. The polypeptide according to the invention may be further modified to contain additional chemical moieties not normally part of the polypeptide. Those derivatized moieties may, e.g., improve the stability, solubility, the biological half life or absorption of the polypeptide. The moieties may also reduce or eliminate any undesirable side effects of the polypeptide and the like. An overview for these moieties can be found, e.g., in Remington's Pharmaceutical Sciences (18th ed., Mack Publishing Co., Easton, PA (1990)). Polyethylene glycol (PEG) is an example for such a chemical moiety which has been used for the preparation of therapeutic polypeptides. The attachment of PEG to polypeptides has been shown to protect them against proteolysis (Sada et al., J. Fermentation Bioengineering 71 (1991), 137-139). Various methods are available for the attachment of certain PEG moieties to polypeptides (for review see: Abuchowski et al., in "Enzymes as Drugs"; Holcerberg and Roberts, eds. (1981), 367-383). Generally, PEG molecules are connected to the polypeptide via a reactive group found on the polypeptide. Amino groups, e.g. on lysines or the amino terminus of the polypeptide are convenient for this attachment among others.

Furthermore, the present invention relates to a binding molecule specifically recognizing the polypeptide of the invention.
The term "specifically recognizing" is meant to refer to the high affinity antibodies or other binding molecules known in the prior art typically have for the target molecule against which they were prepared.
Advantageously, the term "specifically recognizing" refers to a specificity of the binding molecule that allows a distinction between the polypeptide of the invention and related terpene synthase polypeptides, in the sense that the binding molecule does not show a significant cross-reactivity with the latter ones. Said related terpene synthase polypeptides may include the caryophyllene synthases known from dicotyledonous plants and/or other terpene synthases from maize. The person skilled in the art is able to prepare such distinctive binding molecules, for example by selecting non-conserved amino acid stretches, e.g. on the basis of an alignment such as the one shown in Figure 1, and using them as a starting point (e.g. as an antigen in the case of antibodies) for preparing the binding molecule.
The binding molecule of the present invention may be selected form the group consisting of antibodies, affybodies, trinectins, anticalins, aptamers, RNAs, PNAs and the like, whereby antibodies are preferred.

Based on prior art literature, the person skilled in the art is familiar with obtaining specific binding molecules that may be useful in the methods, kits and uses provided herein. These molecules are directed and bind specifically to or specifically label the polypeptide of the invention described herein. Non-limiting examples of suitable binding molecules may be selected from aptamers (Gold, Ann. Rev. Biochem. 64 (1995), 763-797)), aptazymes, RNAi, shRNA, RNAzymes, ribozymes (see e.g., EP-B1 0 291 533, EP-A1 0 321 201, EP-B1 0 360 257), antisense DNA, antisense oligonucleotides, antisense RNA, siRNA, antibodies (Harlow and Lane "Antibodies, A Laboratory Manual", CSH Press, Cold Spring Harbor, 1988), affibodies (Hansson, Immunotechnology 4 (1999), 237-252; Henning, Hum Gene Ther. 13 (2000), 1427-1439), lectins, trinectins (Phylos Inc., Lexington, Massachusetts, USA; Xu, Chem. Biol. 9 (2002), 933), anticalins (EPB1 1 017 814) and the like. In accordance with the present invention, the term "aptamer" means nucleic acid molecules that can specifically bind to target molecules. Aptamers commonly comprise RNA, single stranded DNA, modified RNA or modified DNA molecules. The preparation of aptamers is well known in the art and may involve, inter alia, the use of combinatorial RNA libraries to identify binding sites (Gold (1995), Ann. Rev. Biochem 64 , 763-797).
A preferred binding molecule in the context of the present invention is an antibody specific for the polypeptide of the present invention.
The antibody useful in context of the present invention can be, for example, polyclonal or monoclonal. The term "antibody" also comprises derivatives or fragments thereof which still retain the binding specificity.
The polypeptide according to the invention, its fragments or other derivatives thereof, or cells expressing them can be used as an immunogen to produce antibodies thereto. The present invention in particular also includes chimeric, single chain, and humanized antibodies, as well as Fab fragments, or the product of an Fab expression library. Various procedures known in the art may be used for the production of such antibodies and fragments.
In context of the present invention, the term "antibody" relates to full immunoglobulin molecules as well as to parts of such immunoglobulin molecules substantially retaining binding specificity. Furthermore, the term relates, as discussed above, to modified and/or altered antibody molecules, like chimeric and humanized antibodies. The term also relates to recombinantly or synthetically generated/synthesized antibodies. The term also relates to intact antibodies as well as to antibody fragments thereof, like, separated light and heavy chains, Fab, Fab/c, Fv, Fab', F(ab')₂. The term "antibody" also comprises bifunctional antibodies, trifunctional antibodies and antibody constructs, like single chain Fvs (scFv) or antibody-fusion proteins.

Techniques for the production of antibodies are well known in the art and described, e.g. in Harlow and Lane "Antibodies, A Laboratory Manual", CSH Press, Cold Spring Harbor, 1988. Antibodies directed against a polypeptide according to the present invention can be obtained, e.g., by direct injection of the polypeptide into an animal or by administering the polypeptide to an animal, preferably a non-human animal. The antibody so obtained will then bind the polypeptide itself. In this manner, even a sequence encoding only a fragment of the polypeptide can be used to generate antibodies binding the whole native polypeptide.
Particularly preferred in the context of the present invention are monoclonal antibodies. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Various procedures are known in the art and may be used for the production of such antibodies and/or fragments. Thus, the antibody derivatives can also be produced by peptidomimetics. Further, techniques described for the production of single chain antibodies (see, inter alia, US Patent 4,946,778) can be adapted to produce single chain antibodies specifically recognizing the polypeptide of the invention. Also, transgenic animals may be used to express humanized antibodies to the polypeptide of the invention.
Surface plasmon resonance as employed in the BIAcore system can be used to increase the efficiency of phage antibodies which bind to an epitope of the polypeptide of the invention (Schier, Human Antibodies Hybridomas 7 (1996), 97-105; Malmborg, J. Immunol. Methods 183 (1995), 7-13). Accordingly, also phage antibodies can be used in context of this invention.
Binding molecules according to the invention can be used for detecting the presence, absence or amount of the polypeptide of the invention in a sample, in particular in the framework of methods and uses described herein further below. The binding molecules may furthermore be used for isolating the polypeptide from a biological source material or for detecting the polypeptide in a sample.
Furthermore, the present invention relates to a method for producing a transgenic plant, plant cell or plant tissue comprising the introduction of at least one of the above-described polynucleotides, recombinant nucleic acid molecules or vectors of the invention into the genome of a plant, plant cell or plant tissue.
Preferably, said method comprises (a) the introduction of at least one of said polynucleotides, recombinant nucleic acid molecules or vectors into the genome of a plant cell and regenerating the cell of (a) to a transgenic plant or transgenic plant tissue. Optionally, the method may further comprise step (c) producing progeny from the plants produced in step (b).

In one aspect, the present invention accordingly refers to transgenic plant cells which are genetically engineered with at least one of the polynucleotides, recombinant nucleic acid molecules or vectors of the invention described above or of transgenic plant cells which are obtainable by the aforementioned method for producing a transgenic plant cell.

In a further aspect, the invention relates to transgenic plants or plant tissue comprising plant cells which are genetically engineered with the polynucleotide of the invention or which contain the recombinant nucleic acid molecule or the vector of the invention or to transgenic plants obtainable by the method mentioned above.
Preferably, in the transgenic plant of the invention, the polynucleotide of the invention is expressed at least in one part, i.e. organ, tissue or cell type, of the plant. Preferably, this expression leads to an increase of caryophyllene synthase activity in the cells or tissues which express said polynucleotide. Increase of activity can be detected for instance by measuring the amount of transcript and/or protein in the transformed cell, tissue or plant in comparison to corresponding measurements at non-transformed plant cells, tissue or plants. According to the teachings of the present invention, an increase of the activity of the polypeptide of the invention in transgenic plants leads to an increase of resistance against a root-damaging insect to which a corresponding wild-type plant is susceptible or at least more susceptible.
The term "increased activity" refers to a significant increase of the caryophyllene synthase activity of the polypeptide of the invention in the transgenic plant compared to a corresponding wild-type plant. Preferably, said activity is increased in the transgenic plant by at least 10%, preferably by at least 20%, more preferably by at least 50%, and even more preferred by at least 100% as compared to the corresponding wild-type plant. It is even more preferred that the corresponding wild-type plant shows no detectable caryophyllene synthase activity as opposed to a significant caryophyllene synthase activity observable in the transgenic plant of the invention. Caryophyllene synthase activity may be determined by the use of enzyme assays using a preparation from a plant sample by applying suitable methods known in the art or described herein.
An increase of the activity of the polypeptide of the invention may also be inferred from a significant increase of the amount of corresponding transcript and/or protein present in the transgenic plant. Preferentially, transgenic plants having an increased activity of the polypeptide of the invention may be characterized by an increase of the amount of transcript corresponding to the polynucleotide of the invention by at least 20%, preferably at least 50% and more preferably at least 100% as compared to the corresponding wild-type plant. Likewise, it is preferred that transgenic plants having an increased activity of the polypeptide of the invention may be characterized by an increase of the protein amount of the polypeptide of the invention by at least 20%, preferably at least 50% and more preferably at least 100% as compared to the corresponding wild-type plant. Most preferably, the corresponding wild-type plant shows no detectable amount of such transcript and/or such protein as opposed to significant transcript and/or protein amounts observable in the transgenic plant of the invention.
The polynucleotide introduced into the transgenic plant can in principle be expressed in all or substantially all cells of the plant. However, it is also possible that it is only expressed in certain parts, organs, cell types, tissues etc., provided that expression in the root is ensured. Moreover, it is possible that expression of the polynucleotide only takes place upon induction, at a certain developmental stage or, as it is preferred, upon induction by attack of the root by a root-damaging insect. In a preferred embodiment, the polynucleotide is expressed in those parts of the plant that are exposed to pathogen attack, for example the rhizodermis.
In order to be expressed, the polynucleotide that is introduced into a plant cell is preferably operatively linked to one or more expression control sequences, e.g. a promoter, active in this plant cell. Suitable promoter sequences are known to the skilled person and are described in the pertinent literature.
The promoter may be homologous or heterologous with regard to its origin and/or with regard to the gene to be expressed. Suitable promoters are for instance the promoter of the 35S RNA of the Cauliflower Mosaic Virus (see for instance US-A 5,352,605) and the ubiquitin-promoter (see for instance US-A 5,614,399) which lend themselves to constitutive expression. However, promoters which are only activated at a point in time determined by external influences can also be used (see for instance WO 93/07279). In this connection, promoters of heat shock proteins which permit simple induction may be of particular interest. Likewise, artificial and/or chemically inducible promoters may be used in this context. In one embodiment, promoters which ensure constitutive expression are preferred. However, in another preferred embodiment, the polynucleotide may be operatively linked to a promoter which is inducible upon attack by a root-damaging insect. Even more preferably, this promoter is additionally specific for expression in the root. Accordingly, a particularly preferred promoter is or comprises the regulatory sequence of the present invention, which is described in detail further below.
Moreover, the polynucleotide may be linked to a termination sequence which serves to terminate transcription correctly and to add a poly-A-tail to the transcript which is believed to have a function in the stabilization of the transcripts. Such elements are described in the literature (see for instance Gielen et al., EMBO J. 8 (1989), 23-29) and can be replaced at will.
Furthermore, if needed, polypeptide expression can in principle be targeted to any sub-localization of plant cells (e.g. cytosol, plastids, vacuole, mitochondria) or the plant (e.g. apoplast). In order to achieve the localization in a particular compartment, the coding region to be expressed may be linked to DNA sequences encoding a signal sequence (also called "transit peptide") ensuring localization in the respective compartment. It is evident that these DNA sequences are to be arranged in the same reading frame as the coding region to be expressed. Preferred in connection with the present invention is the expression of the polynucleotide of the invention into the cytosol and/or the endoplasmatic reticulum since this is presumed to be the site of sesquiterpene synthesis in plant cells (Chen, Plant Cell 15 (2003), 481-494).
In order to ensure the location in the plastids, it is conceivable to use one of the following transit peptides: of the plastidic Ferredoxin: NADP+ oxidoreductase (FNR) of spinach which is enclosed in Jansen et al. (Current Genetics 13 (1988), 517-522). In particular, the sequence ranging from nucleotides -171 to 165 of the cDNA sequence disclosed therein can be used which comprises the 5' non-translated region as well as the sequence encoding the transit peptide. Another example is the transit peptide of the waxy protein of maize including the first 34 amino acid residues of the mature waxy protein (Klösgen et al., Mol. Gen. Genet. 217 (1989), 155-161). It is also possible to use this transit peptide without the first 34 amino acids of the mature protein. Furthermore, the signal peptides of the ribulose bisphosphate carboxylase small subunit (Wolter et al., Proc. Natl. Acad. Sci. USA 85 (1988), 846-850; Nawrath et al., Proc. Natl. Acad. Sci. USA 91 (1994), 12760-12764), of the NADP malate dehydrogenase (Gallardo et al., Planta 197 (1995), 324-332), of the glutathione reductase (Creissen et al., Plant J. 8 (1995), 167-175) or of the R1 protein (Lorberth et al. Nature Biotechnology 16, (1998), 473-477) can be used.
In order to ensure the location in the vacuole, it is conceivable to use one of the following transit peptides: the N-terminal sequence (146 amino acids) of the patatin protein (Sonnewald et al., Plant J. 1 (1991), 95-106) or the signal sequences described by Matsuoka and Neuhaus (Journal of Experimental Botany 50 (1999), 165-174); Chrispeels and Raikhel (Cell 68 (1992), 613-616); Matsuoka and Nakamura (Proc. Natl. Acad. Sci. USA 88 (1991), 834-838); Bednarek and Raikhel (Plant Cell 3 (1991), 1195-1206); and Nakamura and Matsuoka (Plant Phys. 101 (1993), 1-5).
In order to ensure the localization in the mitochondria, it is for example conceivable to use the transit peptide described by Braun (EMBO J. 11, (1992), 3219-3227).
In order to ensure the localization in the apoplast, it is conceivable to use one of the following transit peptides: signal sequence of the proteinase inhibitor II-gene (Keil et al., Nucleic Acid Res. 14 (1986), 5641-5650; von Schaewen et al., EMBO J. 9 (1990), 30-33), of the levansucrase gene from Erwinia amylovora (Geier and Geider, Phys. Mol. Plant Pathol. 42 (1993), 387-404), of a fragment of the patatin gene B33 from Solanum tuberosum, which encodes the first 33 amino acids (Rosahl et al., Mol Gen. Genet. 203 (1986), 214-220) or of the one described by Oshima et al. (Nucleic Acid Res. 18 (1990), 181).

The transgenic plants of the invention may, in principle, be plants of any plant species. They may be both monocotyledonous and dicotyledonous plants. Preferably, the plants are useful plants, i.e. commercially important plants, cultivated by man for nutrition or for technical, in particular industrial, purposes. They may be sugar storing and/or starch-storing plants, for instance cereal species (rye, barley, oat, wheat, maize, millet, sago etc.), rice, pea, marrow pea, cassava, sugar cane, sugar beet and potato; tomato, rape, soybean, hemp, flax, sunflower, cow pea or arrowroot, fiber-forming plants (e.g. flax, hemp, cotton), oil-storing plants (e.g. rape, sunflower, soybean) and protein-storing plants (e.g. legumes, cereals, soybeans). The plants within the scope of the invention also include fruit trees, palms and other trees or wooden plants being of economical value such as in forestry. Moreover, the method of the invention relates to forage plants (e.g. forage and pasture grasses, such as alfalfa, clover, ryegrass) and vegetable plants (e.g. tomato, lettuce, chicory) and ornamental plants (e.g. roses, tulips, hyacinths). Preferably, the transgenic plant of the invention is a monocotyledonous plant, more preferably a plant of the Poaceae family, particularly preferably a wheat, barley, oat, rye, rice or sorghum plant. Most preferred are transgenic plants being maize.

According to the provisions of the invention, transgenic plants can be prepared by introducing a polynucleotide into plant cells and regenerating the transformed cells to plants by methods well known to the person skilled in the art.
Methods for the introduction of foreign genes into plants are also well known in the art. These include, for example, the transformation of plant cells or tissues with T-DNA using *Agrobacterium tumefaciens* or *Agrobacterium rhizogenes,* the fusion of protoplasts, direct gene transfer (see, e.g., EP-A 164 575), injection, electroporation, vacuum infiltration, biolistic methods like particle bombardment, pollen-mediated transformation, plant RNA virus-mediated transformation, liposome-mediated transformation, transformation using wounded or enzyme-degraded immature embryos, or wounded or enzyme-degraded embryogenic callus and other methods known in the art. The vectors used in the method of the invention may contain further functional elements, for example "left border"- and "right border"-sequences of the T-DNA of *Agrobacterium* which allow stable integration into the plant genome. Furthermore, methods and vectors are known to the person skilled in the art which permit the generation of marker free transgenic plants, i.e. the selectable or scorable marker gene is lost at a certain stage of plant development or plant breeding. This can be achieved by, for example co-transformation (Lyznik, Plant Mol. Biol. 13 (1989), 151-161; Peng, Plant Mol. Biol. 27 (1995), 91-104) and/or by using systems which utilize enzymes capable of promoting homologous recombination in plants (see, e.g., WO97/08331; Bayley, Plant Mol. Biol. 18 (1992), 353-361); Lloyd, Mol. Gen. Genet. 242 (1994), 653-657; Maeser, Mol. Gen. Genet. 230 (1991), 170-176; Onouchi, Nucl. Acids Res. 19 (1991), 6373-6378). Methods for the preparation of appropriate vectors are described by, e.g., Sambrook and Russell (2001), Molecular Cloning: A Laboratory Manual, CSH Press, Cold Spring Harbor, NY, USA.
Suitable strains of *Agrobacterium tumefaciens* and vectors as well as transformation of *Agrobacteria* and appropriate growth and selection media are well known to those skilled in the art and are described in the prior art (GV3101 (pMK90RK), Koncz, Mol. Gen. Genet. 204 (1986), 383-396; C58C1 (pGV 3850kan), Deblaere, Nucl. Acid Res. 13 (1985), 4777; Bevan, Nucleic. Acid Res. 12(1984), 8711; Koncz, Proc. Natl. Acad. Sci. USA 86 (1989), 8467-8471; Koncz, Plant Mol. Biol. 20 (1992), 963-976; Koncz, Specialized vectors for gene tagging and expression studies. In: Plant Molecular Biology Manual Vol 2, Gelvin and Schilperoort (Eds.), Dordrecht, The Netherlands: Kluwer Academic Publ. (1994), 1-22; EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46; An, EMBO J. 4 (1985), 277-287). Although the use of *Agrobacterium tumefaciens* is preferred in the method of the invention, other *Agrobacterium* strains, such as *Agrobacterium rhizogenes,* may be used, for example if a phenotype conferred by said strain is desired.
Methods for the transformation using biolistic methods are well known to the person skilled in the art; see, e.g., Wan, Plant Physiol. 104 (1994), 37-48; Vasil, Bio/Technology 11 (1993), 1553-1558 and Christou (1996) Trends in Plant Science 1, 423-431. Microinjection can be performed as described in Potrykus and Spangenberg (eds.), Gene Transfer To Plants. Springer Verlag, Berlin, NY (1995).
The transformation of most dicotyledonous plants is possible with the methods described above. But also for the transformation of monocotyledonous plants several successful transformation techniques have been developed. These include the transformation using biolistic methods as, e.g., described above as well as protoplast transformation, electroporation of partially permeabilized cells, introduction of DNA using glass fibers, etc. Also, the transformation of monocotyledonous plants by means of Agrobacterium-based vectors has been described (Chan et al., Plant Mol. Biol. 22 (1993), 491-506; Hiei et al., Plant J. 6 (1994) 271-282; Deng et al, Science in China 33 (1990), 28-34; Wilmink et al, Plant Cell Reports 11 (1992), 76-80; May et al., Bio/Technology 13 (1995), 486-492; Conner and Dormisse, Int. J. Plant Sci. 153 (1992), 550-555; Ritchie et al. Transgenic Res. 2 (1993), 252-265). An alternative system for transforming monocotyledonous plants is the transformation by the biolistic approach (Wan and Lemaux, Plant Physiol. 104 (1994), 37-48; Vasil et al., Bio/Technology 11 (1993), 1553-1558; Ritala et al., Plant Mol. Biol. 24 (1994) 317-325; Spencer et al., Theor. Appl. Genet. 79 (1990), 625-631). The transformation of maize in particular has been repeatedly described in the literature (see for instance WO 95/06128, EP 0 513 849, EP 0 465 875, EP 29 24 35; Fromm et al, Biotechnology 8, (1990), 833-844; Gordon-Kamm et al., Plant Cell 2, (1990), 603-618; Koziel et al., Biotechnology 11 (1993), 194-200; Moroc et al., Theor. Appl. Genet. 80, (1990), 721-726; and, preferably, The Maize Handbook. Eds. M. Freeling and V. Walbot, Springer Verlag New York, 1996). The successful transformation of other types of cereals has also been described for instance of barley (Wan and Lemaux, supra; Ritala et al., supra, Krens et al., Nature 296 (1982), 72-74), wheat (Nehra et al., Plant J. 5 (1994), 285-297) and rice.
The resulting transformed plant cell can then be used to regenerate a transformed plant in a manner known by a skilled person.

In addition, the present invention relates to transgenic plants which show an increased activity of the polypeptide encoded by the polynucleotide the invention compared to a corresponding wild-type plant.
In this context, the "increased activity" complies with the definition stated above and it can be determined accordingly.
Corresponding increases of the activity of the polypeptide of the invention may for instance be achieved by expressing said polynucleotide in cells of a transgenic plant from a heterologous construct for example as described above. However, the state of the art provides further methods for achieving a corresponding increased activity. For example, the endogenous gene encoding the caryophyllene synthase of the invention may be modified accordingly at its natural location, e.g. by homologous recombination. In particular, the promoter of this gene can for instance be altered in a way that promoter activity is enhanced. In the alternative, the coding region of the gene can be modified so that the encoded polypeptide shows an increased activity, e.g. by specifically substituting amino acid residues so as to re-establish the amino acid sequence encoded by an active allele, such as the alleles characterized by the coding sequences of SEQ ID Nos. 1 and 3. Applicable homologous recombination techniques (also known as "in vivo mutagenesis") are known to the person skilled in the art and are described in the literature. One such technique involves the use of a hybrid RNA-DNA oligonucleotide ("chimeroplast") which is introduced into cells by transformation (TIBTECH 15 (1997), 441-447; WO95/15972; Kren, Hepatology 25 (1997), 1462-1468; Cole-Strauss, Science 273 (1996), 1386-1389). Thereby, part of the DNA component of the RNA-DNA oligonucleotide is homologous with the target gene sequence, however, displays in comparison to this sequence a mutation or a heterologous region which is surrounded by the homologous regions. The term "heterologous region" refers to any sequence that can be introduced and which is different from that to be modified. By means of base pairing of the homologous regions with the target sequence followed by a homologous recombination, the mutation or the heterologous region contained in the DNA component of the RNA-DNA oligonucleotide can be transferred to the corresponding gene. By means of in vivo mutagenesis, any part of the gene encoding the caryophyllene synthase of the invention can be modified as long as it results in an increase of the activity of this protein.

In a preferred embodiment, the above-described transgenic plants show, upon an increased activity of the protein encoded by the polynucleotide of the invention, an increased resistance against a root-damaging insect to which a corresponding wild-type plant is more susceptible.
The term "increased resistance" may refer both to an enhancement of a resistance already present in the wild-type plant and to the establishment of a resistance that is not present in the wild-type plant.
Preferably, these transgenic plants contain a polynucleotide as defined above, i.e. a polynucleotide or a recombinant nucleic acid molecule that is introduced in a plant cell and the presence of which in the genome of said plant preferably leads to an increased activity of the caryophyllene synthase of the invention, stably integrated into the genome.

The invention also relates to propagation material of the transgenic plants of the invention, said material comprising plant cells according to the invention. The term "propagation material" comprises those components or parts of the plant which are suitable to produce offspring vegetatively or generatively. Suitable means for vegetative propagation are for instance cuttings, callus cultures, rhizomes or tubers. Other propagation material includes for instance fruits, seeds, seedlings, protoplasts, cell cultures etc. The preferred propagation materials are tubers and seeds.
The invention also relates to harvestable parts of the plants of the invention such as, for instance, fruits, seeds, tubers, rootstocks, leaves or flowers.

In accordance with the above explanations, the invention furthermore relates to a method for conferring resistance or increased resistance against a root-damaging insect to a plant comprising the step of providing a transgenic plant in which the activity of the polypeptide encoded by the above-described polynucleotide of the invention is increased as compared to a corresponding wild-type plant.

Also within the scope of the invention is the use of the polynucleotide, of the recombinant nucleic acid molecule, of the vector, of the host cell, of the polypeptide, of the binding molecule or of the transgenic plant of the invention, said matters being described above in detail, for establishing or enhancing resistance against a root-damaging insect in plants.
In a preferred embodiment of the above-mentioned method and the above-mentioned use, the plants are maize plants and the root-damaging insect is the corn rootworm.

In accordance with another aspect of the present invention, it was discovered that the regulatory sequence of the caryophyllene synthase gene of the invention is inducible upon attack of the root by a root-damaging insect.

Accordingly, the invention further relates to a regulatory sequence which comprises a DNA sequence selected from the group consisting of
(a) the DNA sequence shown in SEQ ID NO: 5 or 6;
(b) fragments of the DNA sequence of (a) being capable of mediating the transcription of a coding sequence operably linked thereto; and
(c) DNA sequences the complementary strand of which hybridizes to the DNA sequence of (a) or (b), wherein said DNA sequence is capable of mediating the transcription of a coding sequence operably linked thereto.

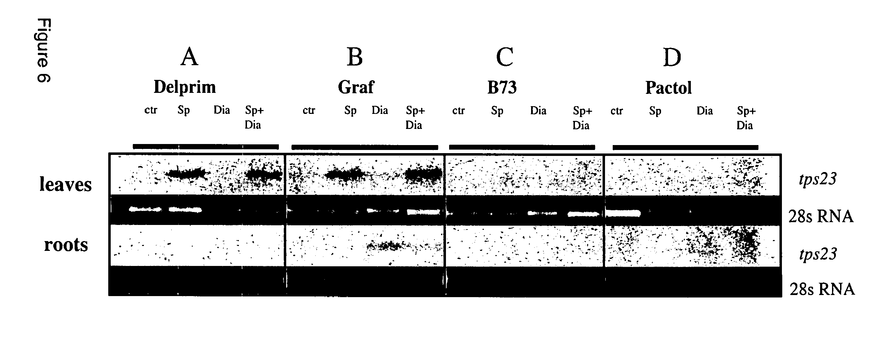

In a preferred embodiment, the regulatory sequence of the invention comprises a promoter sequence from a gene comprising, as its coding region, a caryophyllene synthase-encoding polynucleotide of the invention, as described above.
The regulatory sequence of the present invention is capable of mediating transcription of a coding sequence operably linked thereto, wherein the transcription is inducible upon attack of a plant root by a root-damaging insect as defined above. Preferably, the inducible transcription is tissue-specific, in particular root-specific. The transcription-mediating activity may be assayed by methods known to the person skilled in the art, including in particular the use of promoter-reporter gene constructs in transgenic plants. Examples of suitable reporter genes include luciferase, green fluorescent protein (GFP) and GUS.
In the context with the present invention, the term "regulatory sequence" refers to sequences which influence the specificity and/or level of expression, for example in the sense that they confer inducibility and/or ceii and/or tissue specificity. Such regions can be located upstream of the transcription initiation site, but can also be located downstream of it, e.g., in transcribed but not translated leader sequences.
Accordingly, the term "mediating the transcription of a coding sequence operably linked thereto" refers to the above-mentioned capability of the regulatory sequence to influence the specificity and/or level of expression. The regulatory sequence of the invention may comprise one or more regulatory elements of a promoter or it may be a promoter itself. "Regulatory element" is intended to refer to DNA sequences which on their own have no promoter activity, and require further elements such as CAAT and TATA box in operable linkage so as to form a functional promoter. For example, a regulatory element may become functional when it is combined with a minimal promoter, such as the CaMV 35S, the CHS or other minimal plant promoters well-known in the art.
The term "promoter", within the meaning of the present invention refers to nucleotide sequences necessary for transcription initiation, i.e. RNA polymerase binding, and may also include, for example, the TATA box.
The term "inducible" means that the transcription rate is significantly increased upon attack by a root-damaging insect as compared to the state of the same cell, tissue or organ before that event. Preferably, this increase is at least 2-fold, more preferably at least 5-fold, further preferably at least 10-fold and, most preferably, there is no detectable transcription before the attack in said cell, tissue or organ.
The term "tissue-specific" is intended to refer to a significant higher level of transcription in the tissue in question as compared to other tissues of the same plant. Preferably, the level of transcription in the tissue in question is at least 2-fold, more preferably at least 5-fold and even more preferably at least 10-fold higher than in other tissues of the same plant. Most preferably, there is no detectable transcription in said other tissues as opposed to the tissue in question.
The above-said is correspondingly applicable to organ-specificity and root-specificity in relation to other organs of the same plant.
As is evident from the results reported in appended Example 4 and Figure 6, the TPS23 gene disclosed herein not only shows Diabrotica-induced transcription in the root, but also induced transcription in leaves upon attack by Spodoptera (a caterpillar feeding on leaves. Thus, it is contemplated that the regulatory sequence of the invention may include at least one further inducibility/tissue-specificity characteristic in addition to the root-specific inducibility upon root-damaging insect attack.
The inducibility and tissue-specificity properties can be tested for a given regulatory sequence by methods known to the person skilled in the art. These include the detection of transcripts by way of Northern blot hybridization or RT-PCR as well as the use of promoter-reporter gene constructs in transgenic plants.
Examples of suitable reporter genes include luciferase, green fluorescent protein (GFP) and GUS.

The term "gene" as referred to above relates to caryophyllene synthase genes which comprise as their coding sequence the polynucleotide of the invention encoding said enzyme. In such a gene, the coding sequence may be interrupted by introns. Thus, the regulatory sequence of the invention may be obtained from genes or alleles homologous to the TPS23 gene disclosed herein, said genes or alleles being present in other maize varieties or other plant species.
The caryophyllene synthase genes from which a regulatory sequence according to the invention can be isolated can be selected by examining the inducibility and tissue-specificity properties mentioned above in connection with the regulatory sequence of the invention by applying suitable techniques.

In a preferred embodiment, the regulatory sequence of the invention comprises the sequence shown in SEQ ID NO: 5 or 6. Furthermore, a regulatory sequence of the invention can comprise a fragment of the DNA sequence shown in SEQ ID NO: 5 or 6 which is capable of mediating the transcription of a coding sequence operably linked thereto.
Moreover, the present invention relates to regulatory sequences comprising a DNA sequence the complementary strand of which hybridizes to a DNA sequence as mentioned in sections (a) to (b), above, wherein said DNA sequence is capable of mediating transcription of a coding sequence operably linked thereto.
The present invention also relates to regulatory sequences comprising a DNA a sequence of which has a homology, that is to say a sequence identity, of at least 40%, preferably of at least 50%, more preferably of at least 60%, even more preferably of more than 65%, in particular of at least 70%, especially preferred of at least 80%, in particular of at least 90% and even more preferred of at least 95% when compared to the coding region of the DNA sequence shown in SEQ ID NO: 5 or 6, wherein said DNA sequence is capable of mediating transcription of a coding sequence operably linked thereto.
The invention also relates to polynucleotides comprising a nucleotide sequence which is complementary to the whole or a part of one of the above-mentioned regulatory sequences.
In the context of the present embodiment, the term "hybridization" has the meaning as defined further above. Likewise, the explanations concerning homology and sequence identity stated above apply herein as well.
DNA sequences which hybridize with the regulatory sequences disclosed in connection with the invention can for instance be isolated from genomic libraries of any suitable organism. Preferably, such polynucleotides are from plant origin, particularly preferred from a monocotyledonous plant, more preferably from a plant of the family of Poaceae.
Preferably, the regulatory sequence of the invention is a variant, preferably an ortholog of a regulatory comprising SEQ ID NO: 5 or 6 and may for example comprise a DNA sequence that originates from an agronomically important crop species such as from maize, wheat, barley, oat, rye, rice or sorghum. Alternatively, such polynucleotides can be prepared by genetic engineering or chemical synthesis. The regulatory sequence of the invention may be a naturally occurring promoter or a functional fragment thereof or a regulatory element isolated therefrom capable of mediating transcription of a coding sequence operably linked thereto. Likewise, the regulatory sequence of the invention may be synthetic or may be a chimeric construct, in particular a chimeric promoter, into which for example one or more further functional elements have been introduced following the individual aims and needs.

The invention also relates to polynucleotides (including oligonucleotides) specifically hybridizing with a regulatory sequence of the invention or with the complementary strand thereof. Such polynucleotides have a length of preferably at least 10, in particular at least 15, and particularly preferably of at least 50 nucleotides. Advantageously, their length does not exceed a length of 1000, preferably 500, more preferably 200, still more preferably 100 and most preferably 50 nucleotides. They are characterized in that they specifically hybridize to the regulatory sequences of the invention, that is to say that they do not or only to a very minor extent hybridize to other nucleotide sequences. The oligonucleotides of the invention can be used for instance as primers for amplification techniques such as the PCR reaction or as a hybridization probe to isolate related genes or regulatory sequences. The hybridization conditions and homology values described above in connection with the polynucleotide of the invention encoding a polypeptide having caryophyllene synthase activity may likewise apply in connection with the hybridizing polynucleotides mentioned herein.

In another aspect, the present invention relates to recombinant nucleic acid molecules comprising the regulatory sequence of the invention described above. The definition of the term "recombinant nucleic acid molecule" given above applies herein accordingly.

In a preferred embodiment, the recombinant nucleic acid molecules further comprise a heterologous coding sequence operably linked to the regulatory sequence of the invention. More preferably, these recombinant nucleic acid molecules are expression cassettes. In this context, the term "operatively linked" refers to a linkage between the regulatory sequence and the coding sequence in such a way that expression is achieved under conditions compatible with the regulatory sequence.
Expression comprises transcription of the heterologous coding sequence, preferably into a translatable mRNA. The recombinant nucleic acid molecule may comprise further regulatory elements, as are well known to those skilled in the art, such as enhancers, termination signals, targeting signals and the like. Examples are given above in connection with explanations concerning vectors. Such further expression control sequences may comprise poly-A signals ensuring termination of transcription and stabilization of the transcript, for example, those of the 35S RNA from Cauliflower Mosaic Virus (CaMV) or the nopaline synthase gene from Agrobacterium tumefaciens. Additional regulatory elements may include transcriptional as well as translational enhancers. A plant translational enhancer often used is the CaMV omega sequences. Similarly, the inclusion of an intron (e.g. intron-1 from the shrunken gene of maize) has been shown to increase expression levels by up to 100-fold (Mait, Transgenic Research 6 (1997), 143-156; Ni, Plant Journal 7 (1995), 661-676).

The heterologous coding sequence may be any desired one. Preferentially, it is one which makes use of the specific properties of the regulatory sequence of the invention described above.
In a preferred embodiment, the heterologous coding sequence is a reporter gene. One preferred application of such a recombinant nucleic acid molecules could be to transform plants therewith and to use such plants as biological indicators for the occurrence of root-damaging insects in the soil.
A positive reaction readily visible for example through the dying of the plant or a part thereof as a consequence of reporter gene expression is indicative for the presence of undesirable herbivores in the soil. This would allow to use soil-targeted pesticides very purposefully and in amounts adequate to the pest infestation.

In a preferred embodiment, the present invention relates to recombinant nucleic acid molecules, wherein said heterologous coding sequence encodes an agent active against a herbivore.
In this context, an "agent active against a herbivore" is a polypeptide which, when expressed and brought in contact with a herbivore, especially by uptake of plant material into the digestive tract of the herbivore, reduces the damaging effect of the herbivore on the plant. For example, this agent may be a toxin such as the Bacillus thuringiensis (Bt) toxin, protease inhibitors, natural plant compounds with insect toxicity such as terpenes, phenolics and alkaloids and corresponding enzyme(s) catalyzing the production of such a compound.
According to this embodiment, it is of particular advantage that, due to the expression characteristic of the regulatory sequence of the invention, the expression of the anti-herbivore agent can be limited to the time period when it is required, namely upon herbivore attack. This may meet the interests of a minimization of the presence of agents in crop plants which could potentially present a risk to human health or where unnecessary expression of the agent is undesirable, e.g., for energetic reasons.

In a further preferred embodiment, the heterologous coding sequence comprised in the recombinant nucleic acid molecule of the invention encodes a caryophyllene synthase, preferably from a dicotyledonous plant.
This embodiment could be interesting for applications where, according to the discovery underlying the present invention, resistance against root-damaging insects is aimed at by transforming a target plant with caryophyllene synthase, and where transformation with an endogenous caryophyllene synthase already existing in the plant may cause a co-suppression effect. Accordingly, in the case of maize, it may for example be desirable to transform the plant with a caryophyllene synthase of dicotyledonous origin. In order to achieve an optimal expression profile, the coding sequence encoding a dicotyledonous caryophyllene synthase may then be operably linked to a regulatory sequence of the invention. Corresponding dicotyledonous caryophyllene synthase cDNAs are described in the prior art literature from Arabidopsis, Cucumis and Artemisia (see references mentioned above).

Moreover, the invention relates to vectors, in particular plasmids, cosmids, viruses, bacteriophages and other vectors commonly used in genetic engineering, which contain the above-described regulatory sequence or corresponding recombinant nucleic acid molecule of the invention. In a preferred embodiment of the invention, the vectors of the invention are suitable for the transformation of fungal cells, cells of microorganisms such as yeast or bacterial cells, animal cells or, in particular, plant cells. In a particularly preferred embodiment such vectors are suitable for stable transformation of plants.

In another preferred embodiment, the vectors further comprise a heterologous coding sequence operably linked to said regulatory sequence contained in the vectors.
The above explanations concerning vectors comprising the caryophyllene synthase-encoding polynucleotide of the invention apply herein accordingly.

Additionally, the present invention relates to a method for producing genetically engineered host cells comprising introducing the above-described regulatory sequences and the corresponding recombinant nucleic acid molecules or vectors of the invention into a host cell.

Another embodiment of the invention relates to host cells, in particular prokaryotic or eukaryotic cells, genetically engineered with the above-mentioned regulatory sequences and the corresponding recombinant nucleic acid molecules or vectors of the invention or obtainable by the above-mentioned method for producing genetically engineered host cells, and to cells derived from such transformed cells and containing said regulatory sequence, recombinant nucleic acid molecule or vector of the invention. In a preferred embodiment the host cell is genetically modified in such a way that it contains the regulatory sequence stably integrated into the genome.

Preferentially, the host cell of the invention is a bacterial, yeast, fungus, plant or animal cell.
The transformation of the host cell with a polynucleotide or vector according to the invention can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc.

Furthermore, the invention relates to a method for producing a transgenic plant, plant cell or plant tissue comprising the introduction of at least one of the above-described regulatory sequences of the invention or corresponding recombinant nucleic acid molecules or vectors of the invention into the genome of a plant, plant cell or plant tissue.
Preferably, said method comprises (a) the introduction of at least one of said regulatory sequences, recombinant nucleic acid molecules or vectors into the genome of a plant cell and (b) regenerating the cell of (a) to a transgenic plant or transgenic plant tissue. Optionally, the method may further comprise step (c) producing progeny from the plants produced in step (b).

In one aspect, the invention accordingly refers to transgenic plant cells being genetically engineered with a regulatory sequence of the invention or with corresponding recombinant nucleic acid molecules or vector of the inventions or being obtainable by the aforementioned method for producing a plant cell.

In a further aspect, the invention relates to transgenic plants or plant tissue comprising plant cells which are genetically engineered with the regulatory sequence of the invention or which contain the corresponding recombinant nucleic acid molecule or the vector of the invention or to transgenic plants obtainable by the method mentioned above.

Preferably, the transgenic plant is a maize plant. The above explanations concerning the production of transgenic plant cells, plant tissues and plants, as far as being applicable, are referred to herein as well.

The invention also relates to propagation material of the transgenic plants of the invention being transformed with the regulatory sequence of the invention or corresponding recombinant nucleic acid molecules or vectors, said material comprising plant cells according to the invention. The term "propagation material" comprises those components or parts of the plant which are suitable to produce offspring vegetatively or generatively. Suitable means for vegetative propagation are for instance cuttings, callus cultures, rhizomes or tubers. Other propagation material includes for instance fruits, seeds, seedlings, protoplasts, cell cultures etc. The preferred propagation materials are tubers and seeds.
The invention also relates to harvestable parts of the plants of the invention such as, for instance, fruits, seeds, tubers, rootstocks, leaves or flowers.

In a further embodiment, the present invention relates to the use of the regulatory sequence of the invention or of a corresponding recombinant nucleic acid molecule, vector, host cell or transgenic plant of the invention for establishing or enhancing resistance against a root-damaging insect in plants.
This embodiment is to be seen in the light of the above-stated explanations regarding the usefulness of the regulatory sequence of the invention.
Preferably, said use refers to plants being maize plants and root-damaging insects being the corn rootworm.

In a further aspect, the present invention refers to a method for breeding a plant having a resistance against a root-damaging insect due to the capacity of attracting entomopathogenic nematodes by way of releasing (E)-beta-caryophyllene, said method comprising crossing and/or selfing progenitor lines and selecting for desirable traits, wherein said method is characterized by a selection step for progenitor lines and/or offspring that is/are capable of expressing the polypeptide of the invention.
This embodiment makes use of the contribution of the present invention for marker-assisted breeding approaches. Accordingly, the method of this embodiment comprises conventional plant breeding steps involving the selection for desirable traits such as yield or robustness against biotic or abiotic stress and, additionally, the selection for progenitor lines and/or offspring that show(s) the molecular trait that it expresses the caryophyllene synthase polypeptide of the invention.
According to the breeding method of the invention, the steps of crossing and/or selfing progenitor lines and selecting for desirable traits is understood to include all sorts of conventional breeding techniques that a skilled person commonly applies in order to achieve plants with certain desirable agronomical traits, such as new plant varieties or cultivars, elite lines and, in particular, commercial plant varieties.
Accordingly, the steps of crossing, selfing and selecting may be carried out as is appropriate for the respective plant species. In addition to such steps, the breeding method of the invention may also include one or more further steps, including the application of techniques that are generally considered as being unconventional, such as interspecific crossing or the propagation of a progenitor or pedigree generation by way of non-sexual processes including, for instance, in vitro propagation using cell culture methods as known in the art. It is furthermore envisaged that the breeding method of the invention may also encompass the use of one or more transgenic lines for instance as progenitor lines. Said one or more transgenic lines may be transformed with another trait but the presence of expressible caryophyllene synthase as disclosed above in the context of the present invention.
However, it is preferred that the present method is carried out according to conventional breeding methods, i.e. without the use of genetic engineering. This meets the demand of the consumers for non-genetically engineered crops.
Moreover, the present breeding method may also include the production of hybrid seed as is for example customary practice with crops like maize.
According to the present invention, the step of selecting for progenitor lines or offspring that produce the polypeptide of the invention may be carried out at each suitable stage of the breeding process. For instance, it may be used for screening for progenitor lines with which the breeding is started. Alternatively, it may also be used for selecting those plants of a segregating progeny which are capable of expressing the polypeptide of the invention, in particular when it is known that germplasm introduced into the breeding process at a preceding stage does not contain the caryophyllene synthase trait, as is for example the case with many North American maize lines.
The selection for the capability to express the polypeptide of the invention may be carried out for example by directly determining the presence of the polypeptide of the invention using suitable techniques described in the prior art literature such as antibody-based techniques, e.g. ELISA. Since caryophyllene synthase expression may only occur upon induction, it may be necessary to provide suitable inducing conditions, e. g. by challenging the plant to be assayed with a not-damaging insect, prior to taking a sample for protein analyses. Such a challenge may be substituted by an equivalent treatment as for example mechanical wounding and/or contacting with an elicitor derived from the insect, if such a treatment is known to lead to induction in plants that contain an active caryophyllane synthase allele.
In the alternative, whether or not a plant involved in the breeding process expresses the polypeptide of the invention may likewise be indirectly determined by examining nucleic acid molecules encoding the polypeptide and/or being part of the gene encoding said polypeptide. Thus, the detection may preferably be targeted to the transcript encoding the polypeptide or the corresponding genomic sequences.

In a preferred embodiment of the breeding method of the invention, the molecular selection step comprises the amplification of the caryophyllene synthase-encoding polynucleotide of the invention or of the regulatory sequence of the invention or of a portion of said polynucleotide or regulatory sequence.
The term "amplification" encompasses any suitable technique known to a person skilled in the art by which a relatively small amount of a nucleic acid molecule or a portion thereof can be amplified in vitro to an amount which is susceptible to detection. Preferred amplification techniques include for example PCR, RT-PCR and NASBA.
The amplification product may then be detected by methods known in the art including ethidium bromide agarose gel electrophoresis, acryl amide gel electrophoresis, optionally involving detectable labeling of the product, capillary electrophoresis, DNA chip technology or suitable blot techniques or further techniques being based on the recognition of different conformations of the amplification product.

In a particularly preferred embodiment, said polynucleotide to be detected or portion thereof is RNA and a cDNA obtained from the RNA is amplified
Accordingly, the trait of being capable of expressing the polypeptide of the invention may be indirectly determined by detecting the mRNA encoding it. This is possible due to the result observed when making the present invention that transcriptional regulation is involved in the induction of caryophyllene synthase expression upon attack with a root-damaging insect (see appended Example 4 and Figure 6). In view of this result, it is, however, also necessary to provide inducing conditions, e. g. by challenging the plant to be assayed with a root-damaging insect, prior to taking a sample for RNA analysis. Such a challenge may be substituted by an equivalent treatment as for example mechanical wounding and/or contacting with an elicitor derived from the insect, if such a treatment is known to lead to induction in plants containing an active caryophyllene synthase allele.

In a alternative preferred embodiment of the breeding method of the invention, said selection step comprises the screening for a mutation that imparts the expression of said polypeptide.
This mutation screening may be carried out according to genetic testing methods known to the person skilled in the art. Thereby, the nucleotide and amino acid sequences provided herein (SEQ ID NOs: 1 to 6) may be used as reference sequences for the wild-type state, i.e. for plants being capable of expressing the caryophyllene synthase polypeptide of the invention.
Corresponding mutations may be identified by comparing the sequence of a wild-type allele with that of a mutated allele not being capable of expressing a polypeptide of the invention.
A corresponding deleterious mutation may for example reside in the coding sequence (in particular non-sense or frameshift mutations, but also amino acid substitutions, e.g., at conserved positions essential for protein function), may affect the correct splicing of the primary transcript (e.g. by modifying an exon/intron junction) or may affect the correct transcription or translation of the gene (e.g. by deleterious changes in a regulatory sequence such as the promoter).

It is particularly preferred that, in the above-described breeding method of the invention, the plant is a maize plant, the root-damaging insect is the corn rootworm and the entomopathogenic nematode is Heterorhabditis megidis.

The present invention further relates to a plant obtainable by the above-described breeding method of the invention. This includes preferred forms of products of a breeding method such as seeds or other propagation material.

In accordance with the above explanations, the present invention refers in a further aspect to the use of the polynucleotides of the invention, of the polypeptide of the invention, of the binding molecule of the invention or of the regulatory sequence of the invention for selecting a plant having a resistance against a root-damaging insect. Preferably, the plant is maize and the root-damaging insect is the corn rootworm.

Yet another embodiment of the present invention relates to a kit comprising the above-described polynucleotide specifically hybridizing to a caryophyllene synthase-encoding polynucleotide of the invention or to a regulatory sequence of the invention or a binding molecule of the invention.
The kit may contain further components such a buffers or means for detecting the binding of the hybridizing polynucleotide or binding molecule to the target molecule. If the kit is intended for use in a nucleic acid amplification assay, the kit may contain as further components the materials necessary for carrying out the assay, i.e. suitable buffers and/or enzymes, and the hybridizing polynucleotides may be in the form of primer oligonucleotides, preferably at least one being detectably labeled. Further kits are conceivable in this context, such as kits containing the components for more sophisticated amplification techniques as for example the Taqman™ or the LightCycler™ technology.
The kit of the invention may advantageously be used for carrying out the breeding method of the invention or the corresponding use for selecting a plant having resistance against a root-damaging insect. The parts of the kit of the invention can be packaged individually in vials or in combination in containers or multicontainer units. Manufacture of the kit preferably follows standard procedures which are known to the person skilled in the art.
Moreover, the present invention relates to the use of the transgenic plant of the invention or of propagation material thereof or of a plant obtainable by the breeding method of the invention or propagation material thereof together with entomopathogenic nematodes for growing said plant. Preferably, said transgenic plant or plant is maize, said propagation material is from a maize plant and such entomopathoganic nematode is Heterorhabditis megidis.
It is envisaged that the biological control accomplished by the attraction of entomopathogenic nematodes upon insect attack through the release of caryophyllene may be improved by adding the nematodes to the soil. The nematodes can be cultivated according to methods described in the prior art literature and known to the skilled person. Advantageously, the nematodes are deployed to the field when, or shortly before, attack by root-damaging insects can be expected. Alternatively, the nematodes can be deployed at a time relatively long before attack, for example together with sowing, since nematodes are able to persist for longer periods without nutrition or can feed on other insects present in the soil.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the internet, for example under http://www.ncbi.nim.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.google.de. An overview of patent information in biotechnology and a survey of relevant sources of patent information useful for retrospective searching and for current awareness is given in Berks, TIBTECH 12 (1994), 352-364.

Furthermore, the term "and/or" when occurring herein includes the meaning of "and", "or" and "all or any other combination of the elements connected by said term".

The present invention is further described by reference to the following non-limiting figures and examples.

The Figures show:
- **Figure 1**: shows a comparison of the deduced amino acid sequence of TPS23-Del with (*E*)-β-caryophyllene synthases from other plants (Arabidopsis *thaliana* AtTPS27, *Cucumis sativus* CsCS, *Artemisia annua* QHS1) and two sesquiterpene synthases from maize (TPS10-B73, TPS4-B73). Amino acids identical in all six proteins are marked by black boxes. Amino acids identical in at least four proteins or representing conservative changes are highlighted with gray boxes. The highly conserved DDxxD region is marked with a bar. The accession numbers are: AtTPS27 (AAO85539), CsCS (AAU05952), QHS1 (AAL79181), TPS4-B73 (AAS88571), TPS10-B73 (AAX99146).
- **Figure 2**: shows a dendrogram analysis of the (*E*)-β-caryophyllene synthase TPS23 and other plant terpene synthases. The analysis includes the amino acid sequences of the functionally related (E)-β-caryophyllene synthases AtTPS27 (*Arabidopsis thaliana),* CsCS (*Cucumis sativus),* QHS1 (*Artemisia annua*) and two sesquiterpene synthases from maize, TPS10-B73 and TPS4-B73, which are most closey related but have a different functionality. The dendrogram was constructed using a neighbor joining algorithm and a bootstrap sample of 100. The accession numbers of the sequences are: AtTPS27 (AAO85539), CsCS (AAU05952), QHS1 (AAL79181), TPS4-B73 (AAS88571), TPS10-B73 (AAX99146).

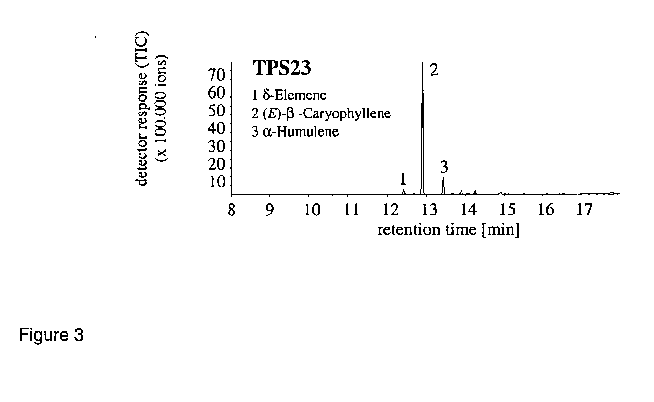

- **Figure 3**: shows an analysis of the sesquiterpene products of TPS23. The enzyme was heterologously expressed in *E. coli,* extracted, partially purified and incubated with the substrate (*E,E*)-FPP. The resulting terpene products were collected with a SPME fibre and analyzed by GC-MS. The products were identified as δ-Elemene (1), (*E*)-β-Caryophyllene (2) and α-Humulene (3) by comparison of their retention times and mass spectra to those of authentic standards.

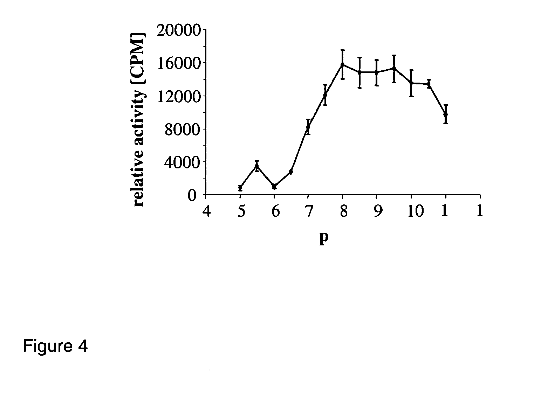

- **Figure 4**: shows that the enzymatic activity of TPS23 is pH dependent. The catalytic activity of the purified enzyme was measured in the presence of 10 mM Mg2+. Different pH values were adjusted with following buffers: pH 5.0 and 5.5: acetate buffer (100 mM); pH 6.0: MES buffer (100 mM); pH 6.5 to 9.5: bis-tris-propane buffer (100 mM); pH 10 to 11: CAPS buffer (10 mM). Means and SE of triplicate assays are shown.

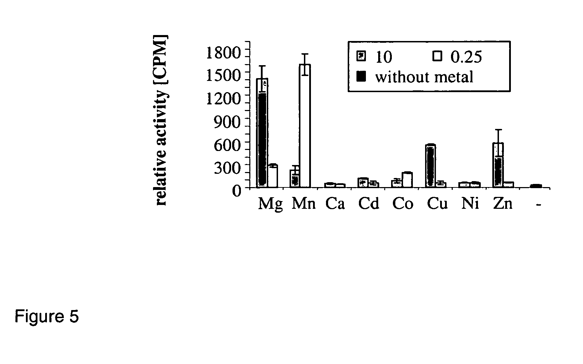

- **Figure 5**: shows that metal cofactors affect the enzymatic activity of TPS23. The catalytic activity of the purified enzyme was measured in the presence of various divalent metal ions at 10 or 0.25 mM. Means and SE of triplicate assays are shown.
- **Figure 6**: shows that the expression of TPS23 is strongly regulated by below- and above-ground herbivory. Transcript levels of *tps23* in different organs of maize cultivars Delprim (A), Graf (B), B73 (C) and Pactol (D) after feeding of *Spodoptera littoralis* (Sp), Diabrotica *virgifera virgifera* (Dia), *S. littorals* and *D. virgifera virgifera* (Sp+Dia) or in undamaged controls (ctr). RNA isolated from 2 weeks old plants was blotted and hybridized with a probe specific for the first 400 bp of *tps23.* Blots were washed under high stringency conditions and exposed to a phosphor imager screen. The bottom panels show the 28s RNA band of the ethidium bromid stained RNA gels as a loading control.

The following Examples serve to further illustrate the invention.

In the Examples the following materials and methods were used.

### 1. Molecular biological techniques

Unless stated otherwise in the Examples, all recombinant DNA techniques are performed according to protocols as described in Sambrook and Russell (2001), Molecular Cloning: A Laboratory Manual, CSH Press, Cold Spring Harbor, NY, USA or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, jointly published by BIOS Scientific Publications Ltd (UK) and Blackwell Scientific Publications (UK).

### 2. Plant and insect material

Plants of the maize (*Zea mays* L.) varieties B73 (KWS seeds, Einbeck, Germany), Delprim (Delley Samen und Pflanzen, Delley, Switzerland), Graf (Landi) and Pactol (Syngenta) were grown in commercially available potting soil in a climate-controlled chamber with a 16 h photoperiod, 1 mmol (m²)⁻¹ s⁻¹ of photosynthetically-active radiation, a temperature cycle of 22 °C/18 °C (day/night) and 65% relative humidity. Twelve to fifteen day old-plants (20-30 cm high, 4-5 expanded leaves) were used in all experiments. Eggs of *Spodoptera littoralis* Boisd. (Lepidoptera: Noctuidae) were obtained from Aventis (Frankfurt, Germany) and were reared on an artificial wheat germ diet (Heliothis mix, Stonefly Industries, Bryan, TX, USA) for about 10-15 d at 22 °C under an illumination of 750 µmol (m²)⁻¹ s⁻¹. For the Spodoptera treatments, three third instar larvae were enclosed on the middle portion of each plant in a cage made out of two halves of a Petri dish (9 cm diameter) with a circle cut out of each side and covered with gauze to allow for ventilation (Röse et al. 1996). Larvae from *Diabrotica virgifera virgifera* LeConte were obtained from the University of Neuchatel, Neuchatel, Switzerland. For the *D. v. virgifera* treatment, each maize plant was subjected to four second instar or third instar larvae for two days.

### 3. cDNA library construction

Ten day-old maize plants of the cultivar Delprim were subjected to herbivory by *Spodoptera littoralis* for four hours. One gram of leaf material was ground in a mortar to a fine powder in liquid nitrogen and added to 10 ml of Trizol Reagent (GIBCO BRL, Rockville, USA). The mixture was treated with a Polytron (Kinematika AG, Switzerland) for one minute and incubated for 3 min on ice. Total RNA was isolated according to manufacturer's instructions. From about 80 µg of total RNA, the mRNA was isolated utilizing poly-T coated ferromagnetic beads (Dynal, Sweden). The mRNA was transcribed into cDNA while constructing a Marathon RACE library according to manufacturer's instructions (Clontech, Palo Alto, CA, USA).

### 4. Isolation of a maize terpene synthase cDNA

Sequences with high similarity to plant terpene synthases were identified in BLAST searches of the TIGR Maize Database (http://maize.tigr.org/). One of these fragments (AZM4_53695; SEQ ID NO: 7) was cloned, sequenced and extended towards the 5' end by the Marathon RACE procedure (Clontech, Palo Alto, CA) with a cDNA library from herbivore-induced leaves of the maize cultivar Delprim. The complete sequence contains an open reading frame of 1644 bp.

### 5. Heterologous expression of terpene synthases

For expression with a N-terminal 8x His tag the ORF of *tps23* was cloned as a *Nco*I-*Eco*RI fragment into the expression vector pHIS8-3. The construct was introduced into the *E. coli* strain BL21 (DE3) and fully sequenced to avoid errors introduced by DNA amplification. Liquid cultures of the bacteria harboring the expression constructs were grown at 37 °C to an OD₆₀₀ of 0.6. Then, isopropyl-β-thiogalactopyranoside was added to a final concentration of 1 mM, and the cultures were incubated for 20 hours at 18 °C. The cells were collected by centrifugation and disrupted by a 4 x 30 s treatment with a sonicator (Bandelin UW2070) in chilled extraction buffer (50 mM Mopso, pH 7.0, with 5 mM MgCl₂, 5 mM sodium ascorbate, 0.5 mM PMSF, 5 mM dithiothreitol and 10% (v/v) glycerol). The cell fragments were removed by centrifugation at 14,000 g and the supernatant was desalted into assay buffer (10 mM Mopso, pH 7.0, 1 mM dithiothreitol, 10% (v/v) glycerol) by passage through a Econopac 10DG column (BioRad, Hercules, CA, USA). For kinetic studies the His-tagged enzyme was further purified on a nickel-nitrilotriacetate agarose column (Qiagen, Heidelberg, Germany) according to manufacturer's instructions.

### 6. Assay for terpene synthase activity

To determine the catalytic activity of the terpene synthase TPS23, enzyme assays containing 50 µl of the bacterial extract and 50 µl assay buffer with 10 µM (*E,E*)-FPP, 10 mM MgCl₂, 0.05 mM MnCl₂, 0.2 mM NaWO₄ and 0.1 mM NaF in a Teflon-sealed, screw-capped 1 ml GC glass vial were performed. A SPME (solid phase microextraction) fiber consisting of 100 µm Polydimethylsiloxane (SUPELCO, Belafonte, PA, USA) was placed into the headspace of the vial for 1 h incubation at 30°C. For analysis of the adsorbed reaction products, the SPME fiber was directly inserted into the injector of the gas chromatograph.
For the determination of metal ion cofactors, *Kₘ* values and effects of pH, an assay containing 1 µM purified TPS23 protein, 10 µM [1-³H](*E,E*)-farnesyl diphosphate (37 GBq mol⁻¹, American Radiolabeled Chemicals, St. Louis, MO, USA) and 10 mM MgCl₂ in 100 µl assay buffer was used. The assay was overlaid with 1 ml pentane to trap volatile products and incubated for 20 min at 30°C. The reaction was stopped by mixing, and 0.5 ml of the pentane layer was taken for measurement of radioactivity by liquid scintillation counting in 2 ml Lipoluma cocktail (Packard Bioscience, Groningen, The Netherlands) using a Packard Tricarb 2300TR liquid scintillation counter (³H efficiency = 61 %).
The pH optimum was determined in buffers from pH 5.0 to pH 11.0. Assay results are reported as the mean of three independent replicate assays, and each experiment was repeated 2-3 times with similar results. The *Kₘ* values were determined using seven substrate concentrations with four repetitions each. The enzyme activity was stable for at least 1 month when stored at -80 °C. The concentration of the purified protein was determined by the method of Bradford (1976) using the BioRad reagent with BSA as standard.

### 7. Gas chromatography

A Hewlett-Packard model 6890 gas chromatograph was employed with the carrier gas He at 1 ml min⁻¹, splitless injection (injector temperature- 220 °C), a Chrompack CP-SIL-5 CB-MS column ((5%-phenyl)-methylpolysiloxane, 25 m x 0.25 mm i.d. x 0.25 µ film thickness, Varian, USA) and a temperature program from 40 °C (3-min hold) at 5 °C min⁻¹ to 240 °C (3 min hold). The coupled mass spectrometer was a Hewlett-Packard model 5973 with a quadrupole mass selective detector, transfer line temperature- 230 °C, source temperature- 230 °C, quadrupole temperature- 150°C, ionization potential- 70 eV and a scan range of 40-350 atomic mass units. Products were identified by comparison of retention times and mass spectra with authentic reference compounds.

### 8. Northern blotting

Plant RNA was prepared with the RNeasy plant mini kit (Qiagen, Hilden, Germany) according the manufacturer's instructions. A 400 bp fragment containing the first two exons of *tps23* was used as a probe, generated by linear PCR with the primer 5'-GAACTTCAAAAATACATCAGA-3' (SEQ ID NO: 10) and the complete ORF as a template. The probe was labeled with 32P-adenosine triphosphate using the Strip-EZ PCR procedure (Ambion, TX, USA). Blotting on a Nytran-Plus nylon membrane (Schleicher & Schuell, Germany), hybridization and washing were carried out following standard procedures. The blots were scanned with a Storm 840 Phosphoimager (Molecular Dynamics, Sunnyvale, CA).

### Example 1: Cloning of the maize terpene synthase gene tps23

A public maize genome database (http://maize.tigr.org/) was screened for sequences with similarity to known terpene synthases. One of the resulting fragments (AZM4_53695; SEQ ID NO: 7) contains the last two exons and the last intron of a putative sesquiterpene synthase gene. The 5' end of this fragment was extended by the Marathon RACE procedure (Clontech, Palo Alto, CA) with a cDNA library from herbivore-induced leaves of the maize cultivar Delprim to obtain the complete open reading frame (SEQ ID NO: 1). The cDNA contains an ORF of 1.644 bp designated as *tps*23-Del that encodes a protein (SEQ ID NO: 2) with a predicted molecular mass of 63.6 kDa. Numerous amino acids throughout its sequence are highly conserved among members of the terpene synthase family (Figure 1). The most characteristic element is an Asp-rich DDxxD motif in the C-terminal part of the protein that is involved in the binding of the divalent metal cofactor (Starks et al., 1997). The gene only has a very low amino acid identity to other maize terpene synthases like TPS10 (40.5%, Schnee et al., 2006) and TPS4 (37.8%, Köllner et al., 2004). No genes with higher sequence identity were found after repeated PCR with maize cDNA and RACE libraries as well as in the maize genomic databases, suggesting that it is a single gene. Also, the caryophyllene synthases AtTPS27, CsCS, QHS1 exhibit a low amino acid identity of 32.9%, 30.3% and 35.1%, respectively. A dendrogram analysis demonstrates that TPS23 is more closely related to functionally unrelated terpene synthases of maize than to terpene synthases of similar function in other plant species, suggesting a convergent evolution of TPS23 (Figure 2).
An allelic variant of the above-mentioned TPS23 sequence has also been isolated from maize cultivar Delprim. Its coding sequence and the deduced amino acid sequence is shown in SEQ ID NO: 3 and 4.

### Example 2: Heterologous expression of TPS23 and product identification

Because it is still not possible to predict the product specificity of a putative terpene synthase from their amino acid sequence, the gene TPS23-Del was cloned with an N-terminal His-tag in the bacterial expression vector pHIS8-3. After expression in *E.coli* BL21 (DE3,) the recombinant protein was extracted, purified and incubated with the potential substrates GPP, FPP and GGPP. The enzyme did not accept GPP or GGPP as a substrate (data not shown), converting only FPP to terpene products (Figure 3). The major product formed from FPP was identified as (*E*)-β-caryophyllene and the two minor products were α-humulene and δ-elemene, as determined by comparison of their retention times and mass spectra to those of authentic standards.

### Example 3: Biochemical characterization of TPS23

To determine the biochemical properties of TPS23-Del, the purified enzyme was incubated with tritium-labeled FPP and the assays performed under the appropriate conditions. The enzyme exhibited a broad catalytic optimum from pH 8.0 to pH 9.5 (Figure 4). The Kₘ value for FPP was 2.4 ± 0.4 µM and the k_{cat} value was 0.0030 ± 0.0002 s⁻¹. Both values are similar to those found for most terpene synthases that have been characterized in other plant species.
A divalent metal ion cofactor is required for enzyme activity (Figure 5). Of the divalent cations tested, Mg²⁺ ions in a concentration of 10 mM and Mn²⁺ ions in a concentration of 0.25 mM gave substantial activities. The Kₘ values were 183 ± 34 µM and 28 ± 6 µM for Mg²⁺ and Mn²⁺, respectively.

### Example 4: Transcript levels of TPS23 are induced by herbivory

In recent publications it was shown that some maize varieties, mostly from the North American maize breeding programs, are not able to produce (*E*)-β-caryophyllene after attack of leaf herbivores (Degen et al., 2004) or root herbivores (Rasmann et al., 2005). To analyze whether TPS23 is involved in the synthesis of (*E*)-β-caryophyllene after herbivory, the transcript level of *tps23* was measured in leaves and roots of two (*E*)-β-caryophyllene-producing and two non-producing maize varieties after attack with larvae of *Spodoptera littoralis* and *Diabrotica virgifera virgifera.* A probe specific for the first two exons of *tps23* was used to detect the level of *tps23* transcript in RNA-blot experiments. Transcripts were detected only in the two European, (*E*)-β-caryophyllene-producing cultivars Delprim and Graf (Figure 6). *Diabrotica* feeding on the roots resulted in transcript accumulation in the roots while herbivory by *Spodoptera* on the leaves led to the accumulation of *tps23* transcripts in the leaves.

The inbred line B73 and the cultivar Pactol are the result of North American breeding programs and do not form detectable levels of *tps23* transcripts. After feeding of larvae, transcript levels of *tps23* are induced in leaves The combined feeding of *D. v. virgifera* on the roots and S. *littoralis* on the leaves does not influence the transcript level of *tps23* in leaves but appears to reduce transcript levels in the roots.

### Example 5: Isolation of TPS23 promoter

The promoter sequences of the TPS23 gene were isolated from genomic DNA prepared from plants of the maize variety Delprim. For isolation of the promoter sequence, the "Genome Walker Kit" of Clontech, Palo Alto, CA, USA was used according to manufacturer's instructions. Briefly, the genomic DNA is cut into fragments by several restriction nucleases and the fragments are ligated to a double stranded adaptor fragment of known sequence. The promoter was isolated by a nested PCR protocol using an adaptor-specific primer and the primer BH12 GACGGATCTTGCCTCATCAGCTGCC (SEQ ID NO: 11) as outer, caryophyllene synthase-specific primer in the first PCR reaction and the primer BH11 GTATACTAGCTAGCTACTCTCCTGC (SEQ ID NO: 12) as inner primer for the second PCR. The procedure was repeated three times to ensure that the promoter sequence is free of PCR artifacts.
The resulting promoter sequences Del-A and Del-B are shown in SEQ ID NOs: 5 and 6, respectively. Note that the depicted DNA sequences contain as the last three residues the start codon ATG of the TPS23 reading frame.

### Cited literature

Bradford, M.M. (1976) A rapid and sensitive method for the quantitation of microgram quantities of protein utilizing the principle of protein-dye binding. Anal. Biochem. 72, 248-254.
Degen T. Dillmann C. Marion-Poll F. Turlings TCJ (2004) High genetic variability of herbivore-induced volatile emission within a broad range of maize inbred lines. Plant Physiology. 135(4):1928-1938
Köllner TG, Schnee C, Gershenzon J, Degenhardt J (2004) Stereospecificity and activity of the husk-expressed terpene synthases TPS4 and TPS5 are dependent on single amino acids in Zea mays. Plant Cell. 16: 1115-1131
Rasman S, Köllner TG, Degenhardt J, Hiltpold I, Töpfer S, Kuhlmann U, Gershenzon J, Turlings TCJ (2005) Recruitment of entomopathogenic nematodes by insect-damaged maize roots. Nature 434: 732-737
Röse USR, Manukian A, Heath RR, Tumlinson JH (1996) Volatile semiochemicals released from undamaged cotton leaves. Plant Physiol 111: 487-495
Schnee C, Köllner TG, Held M, Turlings TCJ, Gershenzon J, Degenhardt J (2006) A maize terpene synthase contributes to a volatile defense signal that attracts natural enemies of maize herbivores. Proc. Natl. Acad. Sci. USA 103(4): 1129-1134
Starks, C.M., Back, K., Chappell, J., and Noel, J.P. (1997) Structural basis for cyclic terpene biosynthesis by tobacco 5-epi-aristolochene synthase. Science 277, 1815-1820.

## Claims

1. A polynucleotide selected from the group consisting of
(a) polynucleotides comprising a nucleotide sequence encoding a polypeptide having the amino acid sequence of SEQ ID NO:2 or 4;
(b) polynucleotides comprising the nucleotide sequence shown in SEQ ID NO:1 or 3;
(c) polynucleotides comprising a nucleotide sequence encoding a fragment of the polypeptide encoded by a polynucleotide of (a) or (b), wherein said nucleotide sequence encodes a protein having caryophyllene synthase activity;
(d) polynucleotides comprising a nucleotide sequence the complementary strand of which hybridizes to the polynucleotide of any one of (a) to (c), wherein said nucleotide sequence encodes a protein having caryophyllene synthase activity; and
(e) polynucleotides comprising a nucleotide sequence that deviates from the nucleotide sequence defined in (d) by the degeneracy of the genetic code.

2. The polynucleotide of claim 1 which is DNA or RNA.

3. A polynucleotide having a length of at least 15 nucleotides which specifically hybridizes with a polynucleotide of claim 1 or 2 or with the complementary strand thereof.

4. A recombinant nucleic acid molecule comprising the polynucleotide of claim 1 or 2.

5. The recombinant nucleic acid molecule of claim 4 further comprising expression control sequences operably linked to said polynucleotide.

6. A vector comprising the polynucleotide of claim 1 or 2 or the recombinant nucleic acid molecule of claim 4 or 5.

7. The vector of claim 6 further comprising expression control sequences operably linked to said polynucleotide.

8. A method for producing genetically engineered host cells comprising introducing the polynucleotide of claim 1 or 2, the recombinant nucleic acid molecule of claim 4 or 5 or the vector of claim 6 or 7 into a host cell.

9. A host cell which is genetically engineered with the polynucleotide of claim 1 or 2, the recombinant nucleic acid molecule of claim 4 or 5 or the vector of claim 6 or 7 or obtainable by the method of claim 8.

10. The host cell of claim 9 which is a bacterial, yeast, fungus, plant or animal cell.

11. A method for the production of a polypeptide encoded by a polynucleotide of claim 1 or 2 in which the host cell of claim 9 or 10 is cultivated under conditions allowing for the expression of the polypeptide and in which the polypeptide is isolated from the cells and/or the culture medium.

12. A polypeptide encoded by the polynucleotide of claim 1 or 2 or obtainable by the method of claim 11.

13. A binding molecule specifically recognizing the polypeptide of claim 12.

14. A method for producing a transgenic plant, plant cell or plant tissue comprising the introduction of the polynucleotide of claim 1 or 2, the recombinant nucleic acid molecule of claim 4 or 5 or the vector of claim 6 or 7 into the genome of a plant, plant cell or plant tissue.

15. A transgenic plant cell which is genetically engineered with the polynucleotide of claim 1 or 2, the recombinant nucleic acid molecule of claim 4 or 5 or the vector of claim 6 or 7 or obtainable by the method of claim 14.

16. A transgenic plant or plant tissue comprising the plant cells of claim 15 or obtainable by the method of claim 14.

17. A transgenic plant which shows an increased activity of the polypeptide encoded by the polynucleotide of claim 1 or 2 compared to a corresponding wild-type plant.

18. The transgenic plant of claim 16 or 17 which is a maize plant.

19. Propagation material or harvestable parts of the transgenic plant of any one of claims 16 to 18 comprising the plant cells of claim 15.

20. A method for conferring resistance or increased resistance against a root-damaging insect to a plant comprising the step of providing a transgenic plant in which the activity of the polypeptide encoded by the polynucleotide of claim 1 or 2 is increased as compared to a corresponding wild-type plant.

21. Use of the polynucleotide of claim 1 or 2, of the recombinant nucleic acid moiecuie of claim 4 or 5, of the vector of claim 6 or 7, of the host cell of claim 9 or 10, of the polypeptide of claim 12, of the binding molecule of claim 13 or of the transgenic plant of any one of claims 16 to 18 for establishing or enhancing resistance against a root-damaging insect in plants.

22. The method of claim 20 or the use of claim 21, wherein the plants are maize plants and the root-damaging insect is the corn rootworm.

23. A regulatory sequence which comprises a DNA sequence selected from the group consisting of
(a) the DNA sequence shown in SEQ ID NO:5 or 6;
(b) fragments of the DNA sequence of (a) being capable of mediating the transcription of a coding sequence operably linked thereto; and
(c) DNA sequences the complementary strand of which hybridizes to the DNA sequence of (a) or (b), wherein said DNA sequence is capable of mediating the transcription of a coding sequence operably linked thereto.

24. The regulatory sequence of claim 23 which comprises a promoter sequence from a gene comprising the polynucleotide of claim 1.

25. A polynucleotide having a length of at least 15 nucleotides which specifically hybridizes with a regulatory sequence of claim 23 or 24 or with the complementary strand thereof.

26. A recombinant nucleic acid molecule comprising the regulatory sequence of claim 23 or 24.

27. The recombinant nucleic acid molecule of claim 26 further comprising a heterologous coding sequence operably linked to said regulatory sequence.

28. The recombinant nucleic acid molecule of claim 27, wherein said coding sequence encodes an agent active against a herbivore.

29. The recombinant nucleic acid molecule of claim 27, wherein said coding sequence encodes a caryophyllene synthase.

30. The recombinant nucleic acid molecule of claim 29, wherein said coding sequence is from a dicotyledonous plant.

31. A vector comprising the regulatory sequence of claim 23 or 24 or the recombinant nucleic acid molecule of any one of claims 26 to 30.

32. The vector of claim 31, further comprising a heterologous coding sequence operably linked to said regulatory sequence.

33. A method for producing genetically engineered host cells comprising introducing the regulatory sequence of claim 23 or 24, the recombinant nucleic acid molecule of any one of claims 26 to 30 or the vector of claim 31 or 32 into a host cell.

34. A host cell which is genetically engineered with the regulatory sequence of claim 23 or 24, the recombinant nucleic acid molecule of any one of claims 26 to 30 or the vector of claim 31 or 32 or obtainable by the method of claim 33.

35. The host cell of claim 34 which is a bacterial, yeast, fungus, plant or animal cell.

36. A method for producing a transgenic plant, plant cell or plant tissue comprising the introduction of the regulatory sequence of claim 23 or 24, the recombinant nucleic acid molecule of any one of claims 26 to 30 or the vector of claim 31 or 32 into the genome of a plant, plant cell or plant tissue.

37. A transgenic plant cell being genetically engineered with a regulatory sequence of claim 23 or 24, the recombinant nucleic acid molecule of any one of claim 26 to 30 or the vector of claim 31 or 32 or obtainable by to the method of claim 36.

38. A transgenic plant or plant tissue comprising the plant cells of claim 37 or obtainable by the method of claim 36.

39. The transgenic plant of claim 38 which is a maize plant.

40. Propagation material or harvestable parts of the transgenic plant of claim 38 or 39 comprising the plant cells of claim 37.

41. Use of the regulatory sequence of claim 23 or 24, of the recombinant nucleic acid molecule of any one of claims 26 to 30, of the vector of claim 31 or 32, of the host cell of claim 34 or 35 or of the transgenic plant of claims 38 or 39 for establishing or enhancing resistance against a root-damaging insect in plants.

42. The use of claim 41, wherein the plants are maize plants and the root-damaging insect is the corn rootworm.

43. A method for breeding a plant having a resistance against a root-damaging insect due to the capacity of attracting entomopathogenic nematodes by way of releasing (E)-beta-caryophyllene, said method comprising crossing and/or selfing progenitor lines and selecting for desirable traits,
wherein said method is **characterized by** a selection step for progenitor lines and/or offspring that is/are capable of expressing the polypeptide of claim 12.

44. The method of claim 43, wherein said selection step comprises the amplification of the polynucleotide of claim 1 or 2 or of the regulatory sequence of claim 23 or 24 or of a portion of said polynucleotide or regulatory sequence.

45. The method of claim 44, wherein said polynucleotide or portion thereof is RNA and a cDNA obtained from said RNA is amplified.

46. The method of claim 43 or 44, wherein said selection step comprises the screening for a mutation that imparts the expression of said polypeptide.

47. The method of any one of claims 43 to 46, wherein the plant is a maize plant, the root-damaging insect is corn rootworm and the entomopathogenic nematode is Heterorhabditis megidis.

48. A plant obtainable by the method of any one of claims 43 to 47.

49. Use of the polynucleotide of any one of claims 1 to 3 and 25, of the polypeptide of claim 12, of the binding molecule of claim 13 or of the regulatory sequence of claim 23 or 24 for selecting a plant having a resistance against a root-damaging insect.

50. The use of claim 49, wherein the plant is maize and the root-damaging insect is corn rootworm.

51. A kit comprising the polynucleotide of claim 3 or 25 or the binding molecule of claim 13.

52. Use of the transgenic plant of any one of claims 16 to 18, 38 and 39 or of the propagation material of claim 40 or the plant of claim 48 together with entompathogenic nematodes for growing said plant.

53. The use of claim 52, wherein said transgenic plant or plant is maize, said propagation material is from a maize plant and said entomopathogenic nematode is Heterorhabditis megidis.


SEQUENCE LISTING

<110>  Max-Planck-Gesellschaft zur Förderung der Wissenschaften
       e.V.

<120>  Caryophyllene synthase gene and uses thereof

<130>  M1495 EP S3

<160>  12

<170>  PatentIn version 3.3

<210>  1
<211>  1644
<212>  DNA
<213>  Zea mays

<220>
<221>  CDS
<222>  (1)..(1641)

<400>  1
atg gca gct gat gag gca aga tcc gtc agc agg ctc cac agt gag gag      48
Met Ala Ala Asp Glu Ala Arg Ser Val Ser Arg Leu His Ser Glu Glu
1               5                   10                  15

gac atg cat ggg aag cac cac tcc act cta tgg ggt gac ttc ttc ctt      96
Asp Met His Gly Lys His His Ser Thr Leu Trp Gly Asp Phe Phe Leu
            20                  25                  30

cac cat gtc cca tgt cgt cca gga cag tat ttg att atg aag gat aac      144
His His Val Pro Cys Arg Pro Gly Gln Tyr Leu Ile Met Lys Asp Asn
        35                  40                  45

gtc gaa ata atg aag gag gag gtg aag aaa atg tta ctg gat gtt gga      192
Val Glu Ile Met Lys Glu Glu Val Lys Lys Met Leu Leu Asp Val Gly
    50                  55                  60

tcc tct gat ttg tct cat aag tta gac tgc att gat aca cta gaa cgg      240
Ser Ser Asp Leu Ser His Lys Leu Asp Cys Ile Asp Thr Leu Glu Arg
65                  70                  75                  80

ctt gga ctg gat tac cac tat acc aag gag att gat gaa tta atg tgc      288
Leu Gly Leu Asp Tyr His Tyr Thr Lys Glu Ile Asp Glu Leu Met Cys
                85                  90                  95

aat gtc ttc gaa gct agg gat caa gat ctt gat ctt act aca aca tca      336
Asn Val Phe Glu Ala Arg Asp Gln Asp Leu Asp Leu Thr Thr Thr Ser
            100                 105                 110

cag ctt ttc tat ttg ctt agg aag cat gga tat cat att tct tct gat      384
Gln Leu Phe Tyr Leu Leu Arg Lys His Gly Tyr His Ile Ser Ser Asp
        115                 120                 125

gta ttt ttg aag ttc aga gat gat aaa gga gat atc gta aca aat gat      432
Val Phe Leu Lys Phe Arg Asp Asp Lys Gly Asp Ile Val Thr Asn Asp
    130                 135                 140

gca agg tgt tta ctg agg atg tac gaa gcg gca cat gtt aga gtg aac 480
Ala Arg Cys Leu Leu Arg Met Tyr Glu Ala Ala His Val Arg Val Asn
145                 150                 155                 160

ggt gaa gaa ata ctt gac aat att ctc att cac acc aag aga caa ctt 528
Gly Glu Glu Ile Leu Asp Asn Ile Leu Ile His Thr Lys Arg Gln Leu
                165                 170                 175

caa tgt att gta gat gat ctc gaa cca aca ctg caa gaa gaa gtt aga 576
Gln Cys Ile Val Asp Asp Leu Glu Pro Thr Leu Gln Glu Glu Val Arg
            180                 185                 190

tat gca ttg gag aca cct ctt ttc aga aga ctc aac aga gta cag gcc 624
Tyr Ala Leu Glu Thr Pro Leu Phe Arg Arg Leu Asn Arg Val Gln Ala
        195                 200                 205

agg caa ttc atc tca aca tac gag aaa agc act acg cgt atc aat atg 672
Arg Gln Phe Ile Ser Thr Tyr Glu Lys Ser Thr Thr Arg Ile Asn Met
    210                 215                 220

ctc ctg gag ttc tca aaa tta gac ttc aac atc ttg ctc acc cta tac 720
Leu Leu Glu Phe Ser Lys Leu Asp Phe Asn Ile Leu Leu Thr Leu Tyr
225                 230                 235                 240

tgt gag gag cta aaa gat ctt acg ctg tgg tgg aaa gag ttc caa gca 768
Cys Glu Glu Leu Lys Asp Leu Thr Leu Trp Trp Lys Glu Phe Gln Ala
                245                 250                 255

cag gct aac acg aca atc tat gca cga gat agg atg gtg gag atg cat 816
Gln Ala Asn Thr Thr Ile Tyr Ala Arg Asp Arg Met Val Glu Met His
            260                 265                 270

ttc tgg atg atg gga gtc ttc ttt gag ccc caa tac tcc tat tca cga 864
Phe Trp Met Met Gly Val Phe Phe Glu Pro Gln Tyr Ser Tyr Ser Arg
        275                 280                 285

aag atg ctc acg cag ttg ttt atg atc gtg tct gta ctc gat gac ttg 912
Lys Met Leu Thr Gln Leu Phe Met Ile Val Ser Val Leu Asp Asp Leu
    290                 295                 300

tat gac agc cat tgc acc aca gag gaa ggc aac gcc ttc act gca gca 960
Tyr Asp Ser His Cys Thr Thr Glu Glu Gly Asn Ala Phe Thr Ala Ala
305                 310                 315                 320

tta caa agg tgg gat gaa gaa ggc gtg gag caa tgc cca aca tac ttg 1008
Leu Gln Arg Trp Asp Glu Glu Gly Val Glu Gln Cys Pro Thr Tyr Leu
                325                 330                 335

agg act ctg tat aca aat ata cgt gcc acc ata aaa gcc atc gag gag 1056
Arg Thr Leu Tyr Thr Asn Ile Arg Ala Thr Ile Lys Ala Ile Glu Glu
            340                 345                 350

gac ttg aat ttt caa aac aac aaa cat gca aag ttg gta aaa gga ctg 1104
Asp Leu Asn Phe Gln Asn Asn Lys His Ala Lys Leu Val Lys Gly Leu
        355                 360                 365

ata atc gac atg gcc atg tgc tat aat gcc gag aca gaa tgg cgt gac 1152
Ile Ile Asp Met Ala Met Cys Tyr Asn Ala Glu Thr Glu Trp Arg Asp
    370                 375                 380

aag aaa tat gtg cca gcc acc gtt gat gaa cat ctg aaa att tca gca     1200
Lys Lys Tyr Val Pro Ala Thr Val Asp Glu His Leu Lys Ile Ser Ala
385                 390                 395                 400

cgc agc agt ggc tgt atg cac ttg gtg agc cag ggg ttc att tcg atg     1248
Arg Ser Ser Gly Cys Met His Leu Val Ser Gln Gly Phe Ile Ser Met
                405                 410                 415

gga gat gtg gca act agc gag gcc ctc gag tgg gcc tct acc tat ccc     1296
Gly Asp Val Ala Thr Ser Glu Ala Leu Glu Trp Ala Ser Thr Tyr Pro
            420                 425                 430

aaa atc gtt cga gct gtc tgt atc att gca cgc ctc gct aat gat ata     1344
Lys Ile Val Arg Ala Val Cys Ile Ile Ala Arg Leu Ala Asn Asp Ile
        435                 440                 445

atg tcg tac aag cga gaa gca tca aac aat act atg gtg tct aca gtg     1392
Met Ser Tyr Lys Arg Glu Ala Ser Asn Asn Thr Met Val Ser Thr Val
    450                 455                 460

caa act tgt gca aaa gaa tac ggc acc acc aca gta gaa cag gcc ata     1440
Gln Thr Cys Ala Lys Glu Tyr Gly Thr Thr Thr Val Glu Gln Ala Ile
465                 470                 475                 480

gaa aag atc aga gag ctg atc gag gaa gcg tgg atg gac atc acc cat     1488
Glu Lys Ile Arg Glu Leu Ile Glu Glu Ala Trp Met Asp Ile Thr His
                485                 490                 495

gag tgc ctc aga cag cca cag ccg aag gct ctt cta gag agg gcg gtc     1536
Glu Cys Leu Arg Gln Pro Gln Pro Lys Ala Leu Leu Glu Arg Ala Val
            500                 505                 510

aac ctg gct cgt aca atg gat ttt ctg tac aaa gat gcg gat gga tac     1584
Asn Leu Ala Arg Thr Met Asp Phe Leu Tyr Lys Asp Ala Asp Gly Tyr
        515                 520                 525

aca gat tca cgg agt atc aaa ggc ata ttg gat tca ttg tat gtg cat     1632
Thr Asp Ser Arg Ser Ile Lys Gly Ile Leu Asp Ser Leu Tyr Val His
    530                 535                 540

cta ata gac taa                                                     1644
Leu Ile Asp
545


<210>  2
<211>  547
<212>  PRT
<213>  Zea mays

<400>  2

Met Ala Ala Asp Glu Ala Arg Ser Val Ser Arg Leu His Ser Glu Glu
1               5                   10                  15


Asp Met His Gly Lys His His Ser Thr Leu Trp Gly Asp Phe Phe Leu
            20                  25                  30

His His Val Pro Cys Arg Pro Gly Gln Tyr Leu Ile Met Lys Asp Asn
        35                  40                  45

Val Glu Ile Met Lys Glu Glu Val Lys Lys Met Leu Leu Asp Val Gly
    50                  55                  60

Ser Ser Asp Leu Ser His Lys Leu Asp Cys Ile Asp Thr Leu Glu Arg
65                  70                  75                  80

Leu Gly Leu Asp Tyr His Tyr Thr Lys Glu Ile Asp Glu Leu Met Cys
                85                  90                  95

Asn Val Phe Glu Ala Arg Asp Gln Asp Leu Asp Leu Thr Thr Thr Ser
            100                 105                 110

Gln Leu Phe Tyr Leu Leu Arg Lys His Gly Tyr His Ile Ser Ser Asp
        115                 120                 125

Val Phe Leu Lys Phe Arg Asp Asp Lys Gly Asp Ile Val Thr Asn Asp
    130                 135                 140

Ala Arg Cys Leu Leu Arg Met Tyr Glu Ala Ala His Val Arg Val Asn
145                 150                 155                 160

Gly Glu Glu Ile Leu Asp Asn Ile Leu Ile His Thr Lys Arg Gln Leu
                165                 170                 175

Gln Cys Ile Val Asp Asp Leu Glu Pro Thr Leu Gln Glu Glu Val Arg
            180                 185                 190

Tyr Ala Leu Glu Thr Pro Leu Phe Arg Arg Leu Asn Arg Val Gln Ala
        195                 200                 205

Arg Gln Phe Ile Ser Thr Tyr Glu Lys Ser Thr Thr Arg Ile Asn Met
    210                 215                 220

Leu Leu Glu Phe Ser Lys Leu Asp Phe Asn Ile Leu Leu Thr Leu Tyr
225                 230                 235                 240

Cys Glu Glu Leu Lys Asp Leu Thr Leu Trp Trp Lys Glu Phe Gln Ala
                245                 250                 255

Gln Ala Asn Thr Thr Ile Tyr Ala Arg Asp Arg Met Val Glu Met His
            260                 265                 270

Phe Trp Met Met Gly Val Phe Phe Glu Pro Gln Tyr Ser Tyr Ser Arg
        275                 280                 285

Lys Met Leu Thr Gln Leu Phe Met Ile Val Ser Val Leu Asp Asp Leu
    290                 295                 300

Tyr Asp Ser His Cys Thr Thr Glu Glu Gly Asn Ala Phe Thr Ala Ala
305                 310                 315                 320

Leu Gln Arg Trp Asp Glu Glu Gly Val Glu Gln Cys Pro Thr Tyr Leu
                325                 330                 335

Arg Thr Leu Tyr Thr Asn Ile Arg Ala Thr Ile Lys Ala Ile Glu Glu
            340                 345                 350

Asp Leu Asn Phe Gln Asn Asn Lys His Ala Lys Leu Val Lys Gly Leu
        355                 360                 365

Ile Ile Asp Met Ala Met Cys Tyr Asn Ala Glu Thr Glu Trp Arg Asp
    370                 375                 380

Lys Lys Tyr Val Pro Ala Thr Val Asp Glu His Leu Lys Ile Ser Ala
385                 390                 395                 400

Arg Ser Ser Gly Cys Met His Leu Val Ser Gln Gly Phe Ile Ser Met
                405                 410                 415

Gly Asp Val Ala Thr Ser Glu Ala Leu Glu Trp Ala Ser Thr Tyr Pro
            420                 425                 430

Lys Ile Val Arg Ala Val Cys Ile Ile Ala Arg Leu Ala Asn Asp Ile
        435                 440                 445

Met Ser Tyr Lys Arg Glu Ala Ser Asn Asn Thr Met Val Ser Thr Val
    450                 455                 460

Gln Thr Cys Ala Lys Glu Tyr Gly Thr Thr Thr Val Glu Gln Ala Ile
465                 470                 475                 480

Glu Lys Ile Arg Glu Leu Ile Glu Glu Ala Trp Met Asp Ile Thr His
                485                 490                 495

Glu Cys Leu Arg Gln Pro Gln Pro Lys Ala Leu Leu Glu Arg Ala Val
            500                 505                 510

Asn Leu Ala Arg Thr Met Asp Phe Leu Tyr Lys Asp Ala Asp Gly Tyr
        515                 520                 525

Thr Asp Ser Arg Ser Ile Lys Gly Ile Leu Asp Ser Leu Tyr Val His
    530                 535                 540

Leu Ile Asp
545

<210>  3
<211>  1644
<212>  DNA
<213>  Zea mays

<220>
<221>  CDS
<222>  (1)..(1641)

<400>  3
atg gca gct gat gag gca aga tcc gtc agc agg ctc cac agt gag gag        48
Met Ala Ala Asp Glu Ala Arg Ser Val Ser Arg Leu His Ser Glu Glu
1               5                   10                  15

gac atg cat ggg aag cac cac tcc act cta tgg ggt gac ttc ttc ctt        96
Asp Met His Gly Lys His His Ser Thr Leu Trp Gly Asp Phe Phe Leu
            20                  25                  30

cac cat gtc cca tgt cgt cca gga cag tat ttg att atg aag gat aac       144
His His Val Pro Cys Arg Pro Gly Gln Tyr Leu Ile Met Lys Asp Asn
        35                  40                  45

gtc gaa ata atg aag gag gag gtg aag aaa atg tta ctg gat gtt gga       192
Val Glu Ile Met Lys Glu Glu Val Lys Lys Met Leu Leu Asp Val Gly
    50                  55                  60

tcc tct gat ttg tct cat aag tta gac tgc att gat aca cta gaa cgg       240
Ser Ser Asp Leu Ser His Lys Leu Asp Cys Ile Asp Thr Leu Glu Arg
65                  70                  75                  80

ctt gga ctg gat tac cac tat acc aag gag att gat gaa tta atg tgc       288
Leu Gly Leu Asp Tyr His Tyr Thr Lys Glu Ile Asp Glu Leu Met Cys
                85                  90                  95

aat gtc ttc gaa gct agg gat caa gat ctt gat ctt act aca aca tca       336
Asn Val Phe Glu Ala Arg Asp Gln Asp Leu Asp Leu Thr Thr Thr Ser
            100                 105                 110

cag ctt ttc tat ttg ctt agg aag cat gga tat cat att tct tct gat       384
Gln Leu Phe Tyr Leu Leu Arg Lys His Gly Tyr His Ile Ser Ser Asp
        115                 120                 125

gta ttt ttg aag ttc aga gat gat aaa gga gat atc gta aca aag gat       432
Val Phe Leu Lys Phe Arg Asp Asp Lys Gly Asp Ile Val Thr Lys Asp
    130                 135                 140

gca agg tgt tta ctg agg atg tac gaa gcg gca cat gtt aga gtg aac       480

Ala Arg Cys Leu Leu Arg Met Tyr Glu Ala Ala His Val Arg Val Asn
145                 150                 155                 160

ggt gaa gaa ata ctt gac aat att ctc att cac acc aag aga caa ctt      528
Gly Glu Glu Ile Leu Asp Asn Ile Leu Ile His Thr Lys Arg Gln Leu
                165                 170                 175

caa tgt att gta gat gat ctc gaa cca aca ctg caa gaa gaa gtt aga      576
Gln Cys Ile Val Asp Asp Leu Glu Pro Thr Leu Gln Glu Glu Val Arg
            180                 185                 190

tat gca ttg gag aca cct ctt ttc aga aga ctc aac aga gta cag gcc      624
Tyr Ala Leu Glu Thr Pro Leu Phe Arg Arg Leu Asn Arg Val Gln Ala
        195                 200                 205

agg caa ttc atc tca aca tac gag aaa agc act acg cgt atc aat atg      672
Arg Gln Phe Ile Ser Thr Tyr Glu Lys Ser Thr Thr Arg Ile Asn Met
    210                 215                 220

ctc ctg gag ttc tca aaa tta gac ttc aac atc ttg ctc acc cta tac      720
Leu Leu Glu Phe Ser Lys Leu Asp Phe Asn Ile Leu Leu Thr Leu Tyr
225                 230                 235                 240

tgt gag gag cta aaa gat ctt acg ctg tgg tgg aaa gag ttc caa gca      768
Cys Glu Glu Leu Lys Asp Leu Thr Leu Trp Trp Lys Glu Phe Gln Ala
                245                 250                 255

cag gct aac acg aca atc tat gca cga gat agg atg gtg gag atg cat      816
Gln Ala Asn Thr Thr Ile Tyr Ala Arg Asp Arg Met Val Glu Met His
            260                 265                 270

ttc tgg atg atg gga gtc ttc ttt gag ccc caa tac tcc tat tca cga      864
Phe Trp Met Met Gly Val Phe Phe Glu Pro Gln Tyr Ser Tyr Ser Arg
        275                 280                 285

aag atg ctc acg cag ttg ttt atg atc gtg tct gta ctc gat gac ttg      912
Lys Met Leu Thr Gln Leu Phe Met Ile Val Ser Val Leu Asp Asp Leu
    290                 295                 300

tat gac agc cat tgc acc aca gag gaa ggc aac gcc ttc act gca gca      960
Tyr Asp Ser His Cys Thr Thr Glu Glu Gly Asn Ala Phe Thr Ala Ala
305                 310                 315                 320

tta caa agg tgg gat gaa gaa ggc gtg gag caa tgc cca aca tac ttg     1008
Leu Gln Arg Trp Asp Glu Glu Gly Val Glu Gln Cys Pro Thr Tyr Leu
                325                 330                 335

agg act ctg tat aca aat ata cgt gcc acc ata aaa gcc atc gag gag     1056
Arg Thr Leu Tyr Thr Asn Ile Arg Ala Thr Ile Lys Ala Ile Glu Glu
            340                 345                 350

gac ttg aat tgt caa aac aac aaa cat gca aag ttg gta aaa gga ctg     1104
Asp Leu Asn Cys Gln Asn Asn Lys His Ala Lys Leu Val Lys Gly Leu
        355                 360                 365

ata atc gac atg gcc atg tgc tat aat gcc gag aca gaa tgg cgt gac     1152
Ile Ile Asp Met Ala Met Cys Tyr Asn Ala Glu Thr Glu Trp Arg Asp
    370                 375                 380

aag aaa tat gtg cca gcc acc gtt gac gaa cat ctg aaa att tca gca     1200

Lys Lys Tyr Val Pro Ala Thr Val Asp Glu His Leu Lys Ile Ser Ala
385                 390                 395                 400

cgc agc agt ggc tgt atg cac ttg gtg agc cag ggg ttc att tcg atg      1248
Arg Ser Ser Gly Cys Met His Leu Val Ser Gln Gly Phe Ile Ser Met
                405                 410                 415

gga gat gtg gca act agc gag gcc ctc gag tgg gcc tct acc tat ccc      1296
Gly Asp Val Ala Thr Ser Glu Ala Leu Glu Trp Ala Ser Thr Tyr Pro
            420                 425                 430

aaa atc gtt cga gct gtc tgt atc att gca cgc ctc gct aat gat ata      1344
Lys Ile Val Arg Ala Val Cys Ile Ile Ala Arg Leu Ala Asn Asp Ile
        435                 440                 445

atg tcg tac aag cga gaa gca tca aac aat act atg gtg tct aca gtg      1392
Met Ser Tyr Lys Arg Glu Ala Ser Asn Asn Thr Met Val Ser Thr Val
    450                 455                 460

caa act tgt gca aaa gaa tac ggc acc acc aca gta gaa cag gcc ata      1440
Gln Thr Cys Ala Lys Glu Tyr Gly Thr Thr Thr Val Glu Gln Ala Ile
465                 470                 475                 480

gaa aag atc aga gag ctg atc gag gaa gcg tgg atg gac atc acc cat      1488
Glu Lys Ile Arg Glu Leu Ile Glu Glu Ala Trp Met Asp Ile Thr His
                485                 490                 495

gag tgc ctc aga cag cca cag ccg aag gct ctt cta gag agg gcg gtc      1536
Glu Cys Leu Arg Gln Pro Gln Pro Lys Ala Leu Leu Glu Arg Ala Val
            500                 505                 510

aac ctg gct cgt aca atg gat ttt ctg tac aaa gat gcg gat gga tac      1584
Asn Leu Ala Arg Thr Met Asp Phe Leu Tyr Lys Asp Ala Asp Gly Tyr
        515                 520                 525

aca gat tca cgg agt atc aaa ggc ata ttg gat tca ttg tat gtg cat      1632
Thr Asp Ser Arg Ser Ile Lys Gly Ile Leu Asp Ser Leu Tyr Val His
    530                 535                 540

cta ata gac taa                                                      1644
Leu Ile Asp
545

<210>  4
<211>  547
<212>  PRT
<213>  Zea mays

<400>  4

Met Ala Ala Asp Glu Ala Arg Ser Val Ser Arg Leu His Ser Glu Glu
1               5                   10                  15

Asp Met His Gly Lys His His Ser Thr Leu Trp Gly Asp Phe Phe Leu
            20                  25                  30

His His Val Pro Cys Arg Pro Gly Gln Tyr Leu Ile Met Lys Asp Asn

35                  40                  45

Val Glu Ile Met Lys Glu Glu Val Lys Lys Met Leu Leu Asp Val Gly
    50                  55                  60

Ser Ser Asp Leu Ser His Lys Leu Asp Cys Ile Asp Thr Leu Glu Arg
65                  70                  75                  80

Leu Gly Leu Asp Tyr His Tyr Thr Lys Glu Ile Asp Glu Leu Met Cys
                85                  90                  95

Asn Val Phe Glu Ala Arg Asp Gln Asp Leu Asp Leu Thr Thr Thr Ser
            100                 105                 110

Gln Leu Phe Tyr Leu Leu Arg Lys His Gly Tyr His Ile Ser Ser Asp
        115                 120                 125

Val Phe Leu Lys Phe Arg Asp Asp Lys Gly Asp Ile Val Thr Lys Asp
    130                 135                 140

Ala Arg Cys Leu Leu Arg Met Tyr Glu Ala Ala His Val Arg Val Asn
145                 150                 155                 160

Gly Glu Glu Ile Leu Asp Asn Ile Leu Ile His Thr Lys Arg Gln Leu
                165                 170                 175

Gln Cys Ile Val Asp Asp Leu Glu Pro Thr Leu Gln Glu Glu Val Arg
            180                 185                 190

Tyr Ala Leu Glu Thr Pro Leu Phe Arg Arg Leu Asn Arg Val Gln Ala
        195                 200                 205

Arg Gln Phe Ile Ser Thr Tyr Glu Lys Ser Thr Thr Arg Ile Asn Met
    210                 215                 220

Leu Leu Glu Phe Ser Lys Leu Asp Phe Asn Ile Leu Leu Thr Leu Tyr
225                 230                 235                 240

Cys Glu Glu Leu Lys Asp Leu Thr Leu Trp Trp Lys Glu Phe Gln Ala
                245                 250                 255

Gln Ala Asn Thr Thr Ile Tyr Ala Arg Asp Arg Met Val Glu Met His
            260                 265                 270

Phe Trp Met Met Gly Val Phe Phe Glu Pro Gln Tyr Ser Tyr Ser Arg

        275                 280                 285

Lys Met Leu Thr Gln Leu Phe Met Ile Val Ser Val Leu Asp Asp Leu
    290                 295                 300

Tyr Asp Ser His Cys Thr Thr Glu Glu Gly Asn Ala Phe Thr Ala Ala
305                 310                 315                 320

Leu Gln Arg Trp Asp Glu Glu Gly Val Glu Gln Cys Pro Thr Tyr Leu
                325                 330                 335

Arg Thr Leu Tyr Thr Asn Ile Arg Ala Thr Ile Lys Ala Ile Glu Glu
            340                 345                 350

Asp Leu Asn Cys Gln Asn Asn Lys His Ala Lys Leu Val Lys Gly Leu
        355                 360                 365

Ile Ile Asp Met Ala Met Cys Tyr Asn Ala Glu Thr Glu Trp Arg Asp
    370                 375                 380

Lys Lys Tyr Val Pro Ala Thr Val Asp Glu His Leu Lys Ile Ser Ala
385                 390                 395                 400

Arg Ser Ser Gly Cys Met His Leu Val Ser Gln Gly Phe Ile Ser Met
                405                 410                 415

Gly Asp Val Ala Thr Ser Glu Ala Leu Glu Trp Ala Ser Thr Tyr Pro
            420                 425                 430

Lys Ile Val Arg Ala Val Cys Ile Ile Ala Arg Leu Ala Asn Asp Ile
        435                 440                 445

Met Ser Tyr Lys Arg Glu Ala Ser Asn Asn Thr Met Val Ser Thr Val
    450                 455                 460

Gln Thr Cys Ala Lys Glu Tyr Gly Thr Thr Thr Val Glu Gln Ala Ile
465                 470                 475                 480

Glu Lys Ile Arg Glu Leu Ile Glu Glu Ala Trp Met Asp Ile Thr His
                485                 490                 495

Glu Cys Leu Arg Gln Pro Gln Pro Lys Ala Leu Leu Glu Arg Ala Val
            500                 505                 510

Asn Leu Ala Arg Thr Met Asp Phe Leu Tyr Lys Asp Ala Asp Gly Tyr

515                 520                 525

Thr Asp Ser Arg Ser Ile Lys Gly Ile Leu Asp Ser Leu Tyr Val His
    530                 535                 540

Leu Ile Asp
545

<210>  5
<211>  1790
<212>  DNA
<213>  Zea mays

<400>  5
   aaccaccaaa attaatactg ggaaaaggtt aaccttattt ccctttcaat tagtctaccc      60
   attttcatga tgtccttctt cgaaatccta ggtggtgtac tacagaagct agatgctatt     120
   cggtctagat tcttttggca aggaggacat cataaaaata aatatagact aggcaaatgg     180
   aaactcattt gtcaacctaa agagctaggt gggctgggag tcgctaatct tgacataaag     240
   aacatttgtt tactcagcac atagcttttt aaattactca atgaagatgg tgtgtggcaa     300
   cgtttattaa gaaacaaata cttgggagac gaatccctaa cacaagctgt cagaaaacca     360
   ggtgattatc atttctgggc tagtctaatg aacatcaagg acaaattcct caagtgggga     420
   tattttaagg tgggaaacgg acaagcaaca agattctggg aagataaatg gcttaataat     480
   tctcctctta gagagtaatt cccaaacctt tttaatatag tgcgcaataa gacctcgctg     540
   gttgcagaag tgctgtcaca tgaaaatcta actctttctt tccggagatc catggttgga     600
   attaagcttg tagaatggca gaacatgata cacttactgg ctacggttaa tctgactccg     660
   gtgagggaca ctttcgtgtg gggagctcat aggaatgggt ctttctcaac ctgatccatg     720
   tactataatt taattagtat cctcatttat aatcgtaacg gtgtgctttg gaaattgaaa     780
   ttacccctta atattaaaat ctttctttcg tacctaggta aggaggtgat cctaaccaaa     840
   gacaatttag gcaaacgtag atggcgagga agtctgaagt gcagtttttg taatcagaac     900
   gagaacatac aacatttttt ctttgattac tacatagcta aaactgtttg gagaattatt     960
   tattttactt taaaaataga ctcacccgtt aatattaatc atattatagg ggattgagaa    1020
   tccaacaaag gttgtatgca caaaaggctg cttctttcta gcgttgcggc tttgttttgg    1080
   tcgatttggc tttgtcgtaa tgatgtggtg tttaatcata accatatacc gtcaactata    1140
   catgtgatct ttagaggtac atattgttca cattttagag actcctgcag atggaagaaa    1200
   cacaacagca aatcttagtc gtgtatcaga ctttggaggt tatggccatg gagttcttca    1260
   cgagtcacgg gtggcggtct aatgcaagga tcgatgccgt ttagtgttta tgatctttta    1320
   ttccatatca tgttttaagt tgaagtttgt ttgatccatg aattcctggg atgatgtaac 1380
   atttgaggtg tatggttgta tgcatgaaaa tgcaaaagtc cggaacgata tttccatttc 1440
   aaaaaaagtc tactggacgg ccaaaccaac attgttgagt ggacgaccta cgatattatt 1500
   aacaattcca tgtgttgcac atgcctgtcg gcagatctat atttaaaaaa aaacgagtgg 1560
   actacgacgc tcagatcctt ccaggaaaaa aaacaatata atgcaaattg agtacgtacg 1620
   tgcccacgtg catcacgagt accactccac gctggctacc tatattaaga acaccatagg 1680
   atccaggtgc ttgccagtgc atgcattgta tatctgcact gctacgccgg tgggcacgta 1740
   cgtgcaacag gcagcaggag agtagctagc tagtatacta tactttcatg 1790

<210> 6
<211> 1789
<212> DNA
<213> Zea mays

<400> 6
aaccaccaaa attaatactg ggaaaaggtt aaccttattt ccctttcaat tagtctaccc 60

attttcatga tgtccttctt cgaaatccta ggtggtgtac tacagaagct agatgctatt 120

cggtccagat tcttttggca aggaggacat cataaaaata aatatagact aggcaaatgg 180

aaactcattt gtcaacctaa agagctaggt gggctgggag tcgctaatct tgacataaag 240

aacatttgtt tactcagcaa atagcttttt aaattactca atgaagatgg tgtgtggcaa 300

cgtttattaa gaaacaaata cttgggagac gaatccctaa cacaagctgt cagaaaacca 360

ggtgattatc atttctgggc tagtctaatg aacatcaagg acaaattcct caagtggaga 420

tattttaagg tgggaaacgg acaagcaaca agattctggg aagataaatg gcttaataat 480

tctcctctta gagagtaatt cccaaacctt tttaatatag tgcgcaataa gacctcgctg 540

gttgcagaag tgctgtcaca tgaaaatcta attctttgtt tccggatatc catggttgga 600

attaagcttg tagaatggca gaacatgata cacttactgg ctacggttaa tctgactccg 660

gtgagggaca ctttcgtgtg gggagctcat aggaatgggt ctttctcaac ccgatccatg 720

tactataatt taattagtat cctcatttat aatcgtaacg gtgtgctttg gaaattgaaa 780

ttacccctta atattaaaat ctttctttcg tacctaggta aggaggtgat cctaaccaaa 840

gacaatttag gcaaacgtag atggcgagga agtctgaagt gcagtttttg taatcagaac 900

gagaacatac aacatttttt ctttgattac tacatagcta aaactgtttg gagaattatt 960

tattttactt taaaaataga cccacccgtt aatattaatc atattatagg ggattgagaa 1020

tccaacaaag gttgtatgca caaaaggctg cttctttcta gcgttgcggc tttgttttgg 1080

tcgatttggc tttgtcgtaa tgatgtggtg tttaatcata accatatacc gtcaattata 1140

catgtgatct ttagaggtac atattgttca gattttagag actcctgcag aaggaagaaa 1200

cacaacagca aatcttagtc gtgtatcaga ctttggaggt tatggccatg gagttcttca 1260

cgagtcacgg gtggcggtct aatgcaagga tcgatgacgt ttagtgttta tgatctttta 1320

ttccatatca tgttttaagt tgaagtttgt ttgatccata aattcctggg atgatgtaac 1380

atttgaggtg tatggttgta ttcatgaaaa tgcaaaagtc cggaacgata tttccatttc 1440

caaaaaagtc tactggacgg ccaaaccaac attgttgagt ggacgaccta cgatattatt 1500

aacaattcca tgtgttgcac atgcctgtcg gcagatctat atttaaaaaa aacgagtgga 1560

ctacgacgct cagatccttc caggaaaaaa aacaatataa tgcaaattga gtacgtacgt 1620

gcccacgtgc atcacgagta ccactccacg ctggctacct atattaagaa caccatagga 1680

tccaggtgct tgccagtgca tgcattgtat atctgcgctg ctacgccggt gggcacgtac 1740

gtgcaacagg cagcaggaga gtagctagct agtatactat actttcatg 1789

<210> 7
<211> 2284
<212> DNA
<213> Zea mays

<400> 7
gacgtacctg agtttgtccc ttaaatcccg ggtgtgacaa gaaaatacag gcaaccaagt 60

agaacttgtt catgcttaat aggttattaa gtgatttgca cgagaagtct ggccgtagtg 120

tgtgtttatt ttagttgtac aaaaatgaat aagtcaaaat tctaaggtat ttaattttta 180

aatagaaaaa ttaatgtagc gaaacaaaaa ataatcactt tttgattgtt gatgcttgaa 240

aatggaaaaa gtattgtgtg gaacacacta agaaataact caattcacgt gccacgtttt 300

tagagacttt gcctcaggtt tccatataaa aaagccacac taatataatt tccaaagttt 360

tggcgcttgg taagggagat tttataatta tattccttta ggaggttggg tccaacccta 420

ggagatttca cccattaagg gcttattcgt tttgctcaat tcatgtggat tgggtaggat 480

tgagtgggtt tcaatcccaa ataagtcaaa atcttttatt tttttaatcc catccaatcc 540

acgtgccata gaaataaccg aacaaaccct aacgtagcat gtagaccggt cggtatcgaa 600

tggtctagtc tcaatgacag tyggttctac cgatcaattg gtggacctac caactgatcg 660

atacggttgg taccgatcag tggagcaatt ggtactaacc actgttttgg atggtttgct 720

tagtaccaac ccaaatcgat tgatactgat tggatttagc aacatgtctt gttataattg 780

gtctacacac gtgtcacgtc atctacctgt ttttaaacta tttgaagtct aaaaaaacaa 840

aagtgttatt tataaattat gtaattttta gyttcacaaa tcatgtaatt tttatatttg 900

cggtttatga attatatagt tttaattatg gtttctgaat cctgtaaatt ttatttgctt 960

aattttgtac tcagatgaat ctttatttca aaacgttgtg tttcctttca aataaaagat 1020

tataaaagaa aaacggatgt ggcgttgagc cgttgaggtg actataggtt atgataaaca 1080

ttgtgtacta gagcattgtg gtagataaaa ctgactatga ttgatagcag ggcaagtggc 1140

acggtctata atggacgtgg cagtagataa gaataaccga ctacacgaaa aaagccgaaa 1200

aaagatatct ggaatatatc agcatgatac cttttgggaa acaaaataca tttgcaaata 1260

ctacaaatta actagataca cacgggtata ttgaattgtt catcctgtgg gttatatagc 1320

caacagtgtt ttccgaacta ttttagtcta ttagatgcac atacaatgaa tccaatatgc 1380

ctttgatact ccgtgaatct gtgtatccat ccgcatcttt gtacagaaaa tccattgtac 1440

gagccaggtt gaccgccctc tctagaagag ccttcggctg tggctgtctg aggcactcat 1500

gggtgatgtc catccacgct tcctcgatca gctctctgat cttttctatg gcctgttcta 1560

ctgtggtggt gccgtattct tttgcacaag tttgcactgt agacaccata gtattgtttg 1620

atgcttctcg ctgcagataa aaaagattct tcaaaaaatt aattaattag aagatatata 1680

tatatatata tatatataca catatatatg gagtaattat tgagttgcac cttgtacgac 1740

attatatcat tagcgaggcg tgcaatgata cagacagctc gaacgatttt gggataggta 1800

gaggcccact cgagggcctc gctagttgcc acatctccca tcgaaatgaa cccctggctc 1860

accaagtgca tacagccact gctgcgtgct gaaattttca gatgttcatc aacggtggct 1920

ggcacatatt tcttgtcacg ccattctgtc tcggcattat agcacatggc catgtcgatt 1980

atctaagatg catgtgaaat tagtgtcaaa taagctcaat aactttataa tctaactata 2040

tactgttcgt tagcaaatgt cattaatttt gtcccgaaca caataacttc tttttataat 2100

ctactacaca tgaaaagaaa atttgttaga actagattag tttagatgtt gcacacttgt 2160

ttgattagca tataataggt aaaaataaag cagtgacaac catattgctt tttttgtcca 2220

aatttaaatg gataacaatg aatctagaca cgtatataat aatacacata catcaaatat 2280

tgca 2284

<210> 8
<211> 920
<212> DNA
<213> Zea mays

<400> 8
catatttatt tggcccaccc ttaatcaaaa ttgattatat cctcctataa ggtctgaaac 60

aactagatct ctctttttct ttctcccttc gtcatcttct ctagccactt tccaactatt 120

ttcaaaatat aatgtaatac caatatagat attactttta tgttattaga tatatagaat 180

caatcaaaca acccaattaa tgtgatgcac acaactagca cctctttttc ttttacaccc 240

tacacatatt tataaggcag ttgacatata tagagggttt tttctagctg atttcacgct 300

atgctatgaa tttgttcata ttggtttctc tagttaattc acactggcct ccatcaacaa 360

tcaggtgtct cttcaagtaa aatatatgac tcgtactgtg ttgtgcaaat gaaaggttta 420

cactatagac aataagagaa taaccaaatt agatgaaata tctttcttgt agtatttgat 480

tatgaaggat aacgtcgaaa taatgaagga ggaggtgaag aaaatgttac tggatgttgg 540

atcctctgat ttgtctcata agttagactg cattgataca ctagaacggc ttggactgga 600

ttaccactat accaaggaga ttgatgaatt aatgtgcaat gtcttcgaag ctagggatca 660

agatcttgat cttactacaa catcacagct tttctatttg cttaggaagc atggatatca 720

tatttcttct ggtaagcttg cagacatgtt ttgctattat agttggcgta tatttattag 780

ttggaacttc aaataacatt aggacttttt ttggtctaca tcttccaccc aagcaacaaa 840

ggggtttttt tactacaaat atattgtggc gatatatata tatatatata tatatatata 900

tatatatata tataatatat 920

<210> 9
<211> 22649
<212> DNA
<213> Zea mays

<220>
<221> misc_feature
<222> (16321)..(16321)
<223> n is a, c, g, or t

<400> 9
   gatcgtcctt cttatcgtcg ttgtgagccc tgccaccttc cgcaggtggc ccggccttgt 60
   gaaagtggcg ccgaagcatg gcgcactccc caagggtgtg cttgacgggc ccctgatgat 120
   aggggcacga ctccttgagc atcttgtcga agagattggc gcctccggga ggtttctgag 180
   ggttcttgta ctcagcggcg gcgacaaggt ccgcgtcggc ggcgtcgcgt ttcgcttgcg 240
   acttcttctt gcctttcttc ttggtgtcgc tctgagtgga cgcctcgggg acatctttcg 300
   gctggcggcc ctggggctgc ttgtccttcc ggaagatggc ctcaaccgct tcctggccag 360
   aggcgaactt ggtggcgatg tccatcagct cgctcgccct ggtgggggtc ttgcgaccca 420
   gcttgctcac caggtcgcgg caggtggtgc cggcgaggaa cgcgccgatg acgtccaagt 480
   cggtgacgtt aggtagctcg gtgcgctgct tcgagaatcg ccggatgtag tcccggagag 540
   actctcccgg ctgctggcgg cagcttcgga gatcccagga gttcccaggg cgcacgtacg 600
   tgccctggaa gttgccgacg aaggcttgga ccaggtcgtc ccagttggag atctgccccg 660
   gaggcaggtg ctccaaccag gctcgagcgg tgtcggagag gaataggggg aggttgcgga 720
   tgatgaggtt gtcatcgtcc gttccaccca gttggcaggc cagccggtag tccgcgagcc 780
   acagttccgg cctcatctcc cccgagtact ttgtgatggt agtcggggtt cggaaccggg 840
   ttgggaacgg cgcccgtcgt atggcccagc tgaaagcctg cggaccgggt ggttcgggcg 900
   agggactccg atcctccccg ctgtcgtagc gtcccccaca cctggggtgg tagcctcggc 960
   gcaccctctc gtcgaggtgg gttcgacggt tgcggtgatg gtgctcgttg ccgaggcgac 1020
   ccggggccgc aggcgctgtg ttgcgcgtgc gcccggtgtg gaccgaggct tcccgcatga 1080
   accgggaagt cgcggtgcga tgctccgagg ggtacccctg ccttcgggag gcagagcttt 1140
   cggcccgtcg gaccgcgaca tcctccagga gattcttgag ctctccctgg atacgccgcc 1200
   cctcggtggt tgatggctcc ggcatcgcgc ggagaagtat tgccgctgca gccaggttct 1260
   ggccgactcc actagaaacc ggtggtggcc ttgccctgac atcgtcggcg atgcggtgct 1320
   ggatgccctg gggtagatga cgcgcttctc cggccggagc ttggcccgcc catttccgcc 1380
   cgatgtcccg gcggaacggc tcaagtgttc ctgctccctc gtcgagcctg gcctgcatct 1440
   cgcggatttg ctcgagctgt gtgtcctgac cccccgcaag gactgggacc acagctagct 1500
   ctcaaaggat gtcaacgcga ggcgcaggcc tagggggatc accgttttcc ggtataccaa 1560
   gatggttgcc ttcgacggga ccccctagat cgacgtggaa acattcacga cttgggccgc 1620
   agtcctcgtc gccgaggctg cggctaccgt cggaacagtc ggaaaggcag tagtcgcatg 1680
   cggtcatgaa gtcctgcatg gcactggggt taccaagtcc ggagaaatcc caacaaaatt 1740
   cgggctcgtc atcttccttg gaatccgagg gcccataggt cgagacggct gtcagccggt 1800
   ctcagggtga ccgcataccg taccccggag ggtttggaca tgcctctatg aaggcttcca 1860
   ccgaagcgaa gtcgcttggt gggtcgaggc tgaatccaga aggcacgaga tgggaatcgg 1920
   tcggtacctc ctggtcgacg ggcggtgacg aagtcgcgtc gggggcagac tacaccgtcg 1980
   tctcgggtac gagggtgacg cccaacaagt cctttgcgag cgtgctggca tcgtccgacc 2040
   gcttggagtt ggcgtgtcgc ggggaaacga cgctcgtctt cgtctcatac tcgagggcga 2100
   cgctcgacgt gtcccccgtt ggggcgccga cgccgtcgac tcgctcgata gccgacgagg 2160
   tgccgcctcc tgcttggcct tggttgcccc gcctcctcct ccgtcggcgg gggaggcgac 2220
   gggacaaacc cgaatgttgc tcttccacca cgtggggaag acgtcgtcga ttccgccgcc 2280
   ggcgggcggg tcgtcggccg ccattatcgc tgtcgcgcgg cggagaaagg agtatcatgt 2340
   tgtagctccc gtcgagggac atgaactcga gactcccaaa acgaagcacc gtcccaggct 2400
   ggaaaggttg ctggagacta cccatctaga gcttgacggg aagctgttcg tcaacacgca 2460
   gcaggcccct acccggcgcg ccaactgtcg gtgtttcgac cccggggggt ccctggaccg 2520
   acgagtaaat tgtcgccgcg tgtcccagcc cagatggggc ggcgcgagac ggagcacgga 2580
   ggggggaaac aaggagacgg gcgtaaaggg gaaacccgcg gccttcgtgt ttgtccagcg 2640
   cccaggtcgg gtgcgcttgc agtagggggt tacaagcgtc cgcgtgggag ggagcgagcg 2700
   gcctgcacgc gtcgtcccgt cctccccgcg tggccaacct tctgtacgag tgccctaggc 2760
   cttcctttta taggcttaag gagagggccc aggtgtacaa taggagatgt agcagtgtgc 2820
   taacgtgtct agcagagagg agctagcgcc ctaagtacat gccgtcgtgg cagccggaga 2880
   ggttttggca ccctgttctt gtgatgtcgt ggccgtcgga ggagcgccgg agccttgcgg 2940
   aaggacagct gtcggggctg tcgagtcctt gctgacgtct ccttgcttcc gtaaggggtt 3000
   gagagccgcc gtcgtcatgg agcacgcggg gcgccatcat tacttgttta ccggggcgag 3060
   ccagatggga cgccggtctt gttccccgta gcctgagcta gctaggggta gggtaatggt 3120
   ggccccccct gtgatgtggt cggtccgagc cctgggtcgg gcgaggcgga ggcttctccg 3180
   aggttgaggt cgagactgtc ttccgaggtc gaggtcgagt ccgagcctcg ggtcgggcga 3240
   ggcggagttc gttgtcttcc gggctgaggc cgagtccgag ccctggggtc gggcgaggcg 3300
   gagttcgtcg tcttccgggg ccgaggccga gtccgagccc tggggtcagg cgaggcggag 3360
   tccgtcgtct tccggagccg aggccgagtc cgagccctgg gtcgggcgag gcagagttcg 3420
   tcgtcttccg gggccgaggc cgagtccgag ccctggggtc gggcgaggcg gagtccgtcg 3480
   tcttccggag ccgaggccga gtccgagccc tgggtcgggc gaggcagagt tcgtcgtccg 3540
   aggccgagac gggggctgag ccctggggtc aggcgaggcg gagcttccta tggcgcccga 3600
   agccggactt ggctgttgtc agcctcactc tgtcgagcgg cacagcagtt ggagcgacgc 3660
   aggcggcgct gtcctcttgt caggcgggtc agtggagcgg cgaagtgact gcggtcactt 3720
   cggctctgtc gactgagggg cgcgcgtcag gataaggtgt caggccatct ttgcattaaa 3780
   tgcttctacg atacggtcgg ttggcgtggc gacctggcca aggttgcttc ttggcgaaga 3840
   ctgggcctcg ggcgagccga aggtgtgtcc attgcttgag ggggccctcg ggcgagacgt 3900
   gaatcctccg gggtcggctg cccttgcccg aggctgggct cgggcgaggc gaggtcatgt 3960
   cccttgagtg gaccaagcct tgacttagac gcacccatca agcctttgca gctttgtgcc 4020
   tatgggggtt accagctgag attaggagtc ttgggggtac ccctaattat ggtccccgac 4080
   agtactcctc cacttgaagg ttcttcaact cggacccgtt gtcgctcctt atcttcttca 4140
   ccttgagctc aaactcattt tgagctctcc ttaggaagcg cttgagggtc ccttgggttt 4200
   cagatttatc ctgcaaaaag aatacccaag tgaagcggga aaaatcatca actataacaa 4260
   gaccatactt acttcctcct atgcttagat aggcgacggg tccgaagagg tccatatgaa 4320
   gcaactccaa aggtcttgat gtggtcatca catttttggt atgatgagag cttcccacct 4380
   gtttacctgc ttgacaagct gcacaaggtc tatctttttt gaaggttacg tttgttagac 4440
   ctatcacgtg ttctcccttt agaagtttgt aaaggttctt catccccata tgtgctaagc 4500
   ggcgatgcca cagccagccc atgctagtct tagcaattaa acatgcatct agaccgacct 4560
   cctcttttgc aaaatcaact aagtagagtt tgtcgtctaa tacaccctta aaagctaatg 4620
   aaccatcact ccttctaaag acggacacat ctacatttgt gaataagcaa ttatatccca 4680
   tattacaaag ctgacttaca gacaataagt tatatccaag cgactcaact aaaaacacat 4740
   tagaaataga gtgctcggat gaaatggcaa tcttccctag tcctttgacc ttgccttgat 4800
   tcccgtcacc gaatatgatt gtcttgggaa tccttgttct tgacgtagga ggtgaacatc 4860
   ttcttctccc ccgtcatgtg gtttgtgcat ccgctgtcga taatccagct tgatcccccg 4920
   gatgcataaa cctgcaaggc aatttaggct tgggtcttag gtacccaact cttgttgggt 4980
   cctacaaggt tagtaacaat ggtcttaggg acccaaatgc aagttttatc tcccttgcat 5040
   tttgccccta atttcctagc aatcacctta ttatcctttc tacaaattgc aaaggaagca 5100
   ttgcaagcat gataaattgt agaaggttca ttagttttcc taggaacatt agcaacattt 5160
   ttcctaggca tatgatgaat gacatttctc ctaggcatat ctctaccatg cacataggaa 5220
   gaacttgaag caaacatggc atttgaaatc atacactcct atgaacattt ctagaatatc 5280
   tcttatcacc ataaatgaaa gcatgattcc tttgactact attagtcata ggagccttcc 5340
   ctttctcctt ggcgggaatg ggagccttat gacttgtgaa gttcttggct tccctcttga 5400
   agccaagccc atccttaatt gaggggtgtc taccaatagt gtaggcatcc cttgcaaatt 5460
   ttagtttgtc agattcattt ttgctagtct taagttgggc attaagacta gccacttcat 5520
   catttaactt tgtaatagag actaggtgtt cactacaagc atcaacatta aaatctttac 5580
   acctattgca aattacaaca tgatctacac aagagttaga tctatttgct acttctagtt 5640
   tagcatttaa atcatcatta acacttttta aactagaaat gttctcatgg caagtagata 5700
   gttcacaaga aagcattcat tccttttaac ttctaaagca agagattttt gtgcctctac 5760
   aaatttatca tgttcttcat acaaaagatc ctcttgcttt tctaacaatc tattcttatc 5820
   attcaaggca tcaattaatt cattaatctt atcaatctta gttctatcta agcctttgaa 5880
   caaactagca taatctattt catcatcgct agattcatca tcactagaag aagcataagt 5940
   agtctttcaa gtgtttacct tcttctcctt tgccatgagg caagtgtgat gctcgttggg 6000
   gaagagggac gatttgttga aggccgaggc tgcgagtcct tcgttgtcgg agtcggacga 6060
   cgaacaatcc aagtcccact ccttgccaag gtgtgcctca cccttagcct tcttgtaagc 6120
   cttcttcttt tccctcttct tctcttgttc ctggtcacta tcattatcgg gacaattagc 6180
   gataaaatga ccaatcttac cacatttgaa gcatgagcac ttcccctttg tcttgttctt 6240
   gttgggatga tccttgtgac cccttagcgc cgtcttgaaa cgcttgatga tgagggccat 6300
   ctcttcctca ttgagtcccg ccgcctcaac ttgtgccacc ttgctaggta gcacttcctt 6360
   gctccttgtt gctttgagag caacggtttg aggctcgtgg attgggccat tcaacgcatc 6420
   atcaacgtat cttgcctcct tgatcatcat ccgcccgctt acaaacttcc caagtatttc 6480
   ttcgggcgtc atcttggtgt acctaggatt ctcacgaata ttgtttacag gatgtggatc 6540
   aaggacagtg aaagacctta gcattaggcg gacgacgtcg tggtccgtcc atcgcgtgct 6600
   tccatagctc cttattttgt tgacaagggt cttgagccgg ttgtacgttt gggttggctc 6660
   ctctgccctg atcatcgcga accttcctag ttcgccttcc accaactcca tcttggtgag 6720
   catggtgacg tcgttcccct catgcgagat cttgagggtg tgccagatct gcttggcgtt 6780
   atccaagccg ctcaccttat ggtattcatc cctgcacaat gatgctagaa gaacagtagt 6840
   agcttgtgca tttttgtgaa tttgctcatt gataaatatg ggactatcag tactatcaaa 6900
   ttgcattcca ctttctacta tctcccatat gcttggatgg agagagaaca agtgactacg 6960
   cattttgtga ctccaaaatc cgtagtcctc tccgtcaaag tgaggaggtt taccaagtgg 7020
   aatggagagt aaatgagcat tggtactttg aggaatacga gagtaatcga aagaaaaatt 7080
   agagttaact gtcttctttt tctcgtagtc attgtcgtcg ttgtcctttt gggaagaaga 7140
   ggactcgtcg ctgtcgtcgt agtagacgat cttcttgatg cgccttttct tcttcccttc 7200
   cttcttcttt cgactcgagc cggagtcagt agctttgtcg tccctcggct cgttgaagat 7260
   ggtctccttc tccttatcat tgaccaccat cccctttccc ttaggatcca tcccttcggg 7320
   cgattagtcc ctttcttgaa gagaacgact ctgataccaa ttgagagcac ctagaggggg 7380
   ggggtgaata ggtgatcctg taaaaacttg aaacttaatg ccacaaaact tgattaggag 7440
   tcagcacaat aaagccaagt ggctagagag aagtgcttgc aagacacgat aacaacaaga 7500
   aaatcaacac agataggcac agtggtttat cctatggttc ggccaagttc aacacttgcc 7560
   tactccacgt tgtggcgtcc caacggacga gggttgcaat caacccctct caagcggtcc 7620
   aaagacccac ttgaatacca cggtgttttg ctttgtttac tatatcccgc ttgcgaggaa 7680
   tatccacaac ttggagcctc tcgcccttac aatttgatgt tcacaaagaa gcacagaagt 7740
   aaggctggga tgagcaacgc acgcaagaca caaaatcaga gcacaaacat gcacacaagt 7800
   cacaactcga gctcacaaca caacccaaag agttctctac tcaaatggag ctctagttgc 7860
   tatcacaaag aatcgaatgc gcggaattgg agtcttggtg cttaggaatg cttagagaat 7920
   gcttggtctc ttcctccatg cgcctagggg tcccttttat agccccaagg cagctaggag 7980
   ccattgagag cattctagga aggcaattct tgccttctgt cgcctggcgc accggccagt 8040
   ccggtgcacc accggacact gtccggcgcg gatttctttc ctattttggc acagccgacc 8100
   gttggatatt cagagccatt ggcgcaccgg acactgtccg gtgccccctt ctgaccgttg 8160
   gctctgccac gcgtcgcgcg cggattacgc ggccgaccgt tggcccggcc gactgttggc 8220
   tcaccggaca gtccggtgca tcatcggaca gtctggtgca tcatcagata gtccggtgat 8280
   ttatagtcgt acgccgccga cgaaacccga gagcagctag ttcgccagag ccagcctggc 8340
   gcaccggaca ctgtccggtg caccaccgga cagtccggtg cacccagact acacagagtc 8400
   ttggctgctc agccaagtct tttccaaatt ggtcttttcc tgtttctagc acttagacac 8460
   aatacattag tctccaaaac aatgtactaa gtcttagaaa cataccttta ctcttgattt 8520
   gcacttctta agtctttggc acaaatatta ctcaaggcat ttgtgtgtag cacttaatca 8580
   ccaaaatctt atagaaatgt cccaagggta catttccctt tcacatatca ctaggcatca 8640
   aggtactaat ggtatatgat gactcgctag tagtcatcag ccaggtgaac aaagactggg 8700
   attgctccat agactcattg gacaactact atgccacggt gcgaaagctg gaggacaaat 8760
   tcgaaggtct agaattccat cacatggaaa gggatcacaa tgcagcagca gatgcactat 8820
   cgaaactagg gtctagtcgg gcccaggttc cacccagagt ttctgttcag gaggtgcagc 8880
   aaccaagcat cgcagcaggg cacgctaagg aatgcaacac gttagaccag gtgaagatag 8940
   accccaatga ctggagggag ccgattatca gatacataaa aatgaggagg agccagacga 9000
   taaggccaca tcagagcaca ttgcaatgca atcagctcac tataccatca tagggggtct 9060
   gctatacagg agaggtgcag gaggagtcct catgagatgc attcactcag ctacggggag 9120
   gcaactgctg gaggaaatcc atgctgggca gtgtggaata cacgcaacat caaagacctt 9180
   cgtagaaaag gctttcaggt catgattcta ttgaccaaca gcaaagaaag atacagcgga 9240
   cttagtccag aagtgcgaag catgctagtt cctattaaag aaacaacact tacccgtcca 9300
   acatctgcaa actatccctg taatcgggcc ttttgcatgc tgggggctgg atatgatcag 9360
   gccattcaag aaagctcagg gaggatacac tcatatgctc gtcgccatcg ataaatttac 9420
   aaagttgata gagtacaaac caatcgcctc tttgacttta gcaaaagcac tggagttcat 9480
   tcaataaata atgttcagat tcaggatacc caataggatc atcacagacc tgggatctaa 9540
   cttcactagc tcagggttct ttggtttcta tgagcagagg agcatctaga tcaagtatgc 9600
   attagtggca cacccgagag ctaacgggca ggtagaaaga gccaacagga tgataattga 9660
   tgcactcaag aagaaagtat tcgacaagag tgaaaagctc ataagcaaat ggatcaggga 9720
   actactgtat gtagtctgga gtctacggac ccaacctagc cgggccctgc atgggaacac 9780
   tccattcttc atggtatacg gatcagaagc agtgttgcta gttgatttga tttttggagc 9840
   acccaggttg acattcgaaa gcatagcaga agcggaagca accagactag aggatgttga 9900
   catattagaa gaagagcgtt tgaatgtcgt catccaatcg gccaggtatc agcagactct 9960
   gaggcgctac cacgacaaag cggtgcgaca ccgatccttt gcagtaggag acttggtact 10020
   ctgtcgcgta ttgacaggag aaggacgaca caaactatca ttgccaatga taggcctttc 10080
   atcgtcgcag aagtcactcg gcctaggtcc taccggctca cccagatgga cggaactcaa 10140
   gtaggaaact catggaatat tgagcatctc aagaaatttt acccatagtg atacaacaaa 10200
   ctccggttgg tgagctacac attgtaagcc agagatctca ttatcaataa atcttttgtg 10260
   ttcgtccact tgcaaacaaa ccctcaaata gtgatcggct taagtcggaa acgccttaag 10320
   ctgacgcagc tcgcatacct gacttagggt gattactata cactgctaac ggagcaatcg 10380
   gcttaagtca gaaatgcctt aagtcggtta gacattctca cgcatacatg acttagggtg 10440
   attactatac cctgctaacg gagtaatcaa cttaagtcga aaacgcctta agtcggtgcg 10500
   acatgctcac gcatacctga cttggggtga ttactatgct ctgctatcgg agcaatcgac 10560
   ctaagtcgga aacgccttaa gtcggcgaga catgcacatg catacatgac ttaggatgat 10620
   tactataccc tgctagcgca gcagccggct taagtcggaa cgccttaagt cggtgtgacg 10680
   tgctctcgct tacataactt agggttacta tgccccatca atgcatcaat cggcttaagt 10740
   tggaagcgcc ttaagtcgga gcaacatgat caattagaca acaaagagct tagggcacgt 10800
   accataccct actcacactc aaagggagaa taactcaacg ctcaaaaaac cgctgaaaaa 10860
   tatcatctca atgaaaaacg ttttctgttt gaacaaatca atgtttgctt tcactctcct 10920
   attactgtgc aggagcatcc tcgcgaggaa ccttatctac aggatcacca gaagtagaag 10980
   gattactcga agtagccgac acatcaagta catcagctac aatatcatta ggcatcacaa 11040
   agtcaggcct ctttattagg atccggccag cccgaatggc cttatccatc gctttgtcac 11100
   accacgcctt catgatagaa cggtcaacct ctaagacctt gatatcacta cgggcagcct 11160
   gcgcatcgcg acgagcaacc tctagctcct gcgaaacaag tttcttcgag gcatgaagct 11220
   ctttgatgat ggaatctttt gaagacaatg acctgacaag tcgtgatacc tcgttctggg 11280
   agattttcaa ctcatctcta tggcgctcga gctctgactt agtaaagcgg tggctcctct 11340
   ccaaggtggt gagggagacg ctagcatcac ggtacaattt gtccaaacag ttccgagaat 11400
   caagaagttt ctgcatctcc tcctacagat aagaagaact tgtgggtaaa gatgtgcaac 11460
   ctctgtagag tgtaaaactg gtatatcagc cgtgctcacg gtcatgagcg gctcggaccc 11520
   tcatatgagt aatttatgga gttaaactta atttgccaga tgcattgcat tatgtgtttt 11580
   attatattaa tgatgtctta ttttattggg ttggtataca cttatactta gtaattgcta 11640
   ataaaatttg accaacttat taaaataaat gctcagctat aaccattatt tgttgattag 11700
   ccttacacta tacatgagcc acatcataag tgagttcatg cacattattc cccacacttg 11760
   ttgaacgatg aacttttgtg agctcactgt tgcgataccc ccccaggtga agagcaggtg 11820
   gccccaaagg aggactacta tgacaagttc gacgaggtct aggtagcatc tcccagtaag 11880
   cttatgacgc caagggataa taatgatatt agtttgttct atttattatc agttattttg 11940
   taagacattt tgttatataa taaagattgt gacattcgtc tctatacacc gagtcattat 12000
   atgtgttgat cttgtttggc gcacatatga gacgtacctg agtttgtccc ttaaatccgg 12060
   gtgtgacaag aaaatacagg caaccaagta gaacttgttc atgcttaata ggttattaag 12120
   tgatttgcac gagaagtctg gccgtagtgt gtgtttattt tagttgtaca aaaatgaata 12180
   agtcaaaatt ctaaggtatt taatttttaa atagaaaaat taatgtagcg aaacaaaaaa 12240
   taatcacttt ttgattgttg atgcttgaaa atggaaaaag tattgtgtgg aacacactaa 12300
   gaaataactc aattcacgtg ccacgttttt agagactttg cctcaggttt ccatataaaa 12360
   aagccacact aatataattt ccaaagtttt ggcgcttggt aagggagatt ttataattat 12420
   attcctttag gaggttgggt ccaaccctag gagatttcac ccattaaggg cttattcgtt 12480
   ttgctcaatt catgtggatt gggtaggatt gagtgggttt caatcccaaa taagtcaaaa 12540
   tcttttattt ttttaatccc atccaatcca cgtgccatag aaataaccga acaaacccta 12600
   acgtagcatg tagaccggtc ggtatcgaat ggtctagtct caatgacagt cggttctacc 12660
   gatcaattgg tggacctacc aactgatcga tacggttggt accgatcagt ggagcaattg 12720
   gtactaacca ctgttttgga tggtttgctt agtaccaacc caaatcgatt gatactgatt 12780
   ggatttagca acatgtcttg ttataattgg tctacacacg tgtcacgtca tctacctgtt 12840
   tttaaactat ttgaagtcta aaaaaacaaa agtgttattt ataaattatg taatttttag 12900
   cttcacaaat catgtaattt ttatatttgc ggtttatgaa ttatatagtt ttaattatgg 12960
   tttctgaatc ctgtaaattt tatttgctta attttgtact cagatgaatc tttatttcaa 13020
   aacgttgtgt ttcctttcaa ataaaagatt ataaaagaaa aacggatgtg gcgttgagcc 13080
   gttgaggtga ctataggtta tgataaacat tgtgtactag agcattgtgg tagataaaac 13140
   tgactatgat tgatagcagg gcaagtggca cggtctataa tggacgtggc agtagataag 13200
   aataaccgac tacacgaaaa aagccgaaaa aagatatctg gaatatatca gcatgatacc 13260
   ttttgggaaa caaaatacat ttgcaaatac tacaaattaa ctagatacac acgggtatat 13320
   tgaattgttc atcctgtggg ttatatagcc aacagtgttt tccgaactat tttagtctat 13380
   tagatgcaca tacaatgaat ccaatatgcc tttgatactc cgtgaatctg tgtatccatc 13440
   cgcatctttg tacagaaaat ccattgtacg agccaggttg accgccctct ctagaagagc 13500
   cttcggctgt ggctgtctga ggcactcatg ggtgatgtcc atccacgctt cctcgatcag 13560
   ctctctgatc ttttctatgg cctgttctac tgtggtggtg ccgtattctt ttgcacaagt 13620
   ttgcactgta gacaccatag tattgtttga tgcttctcgc tgcagataaa aaagattctt 13680
   caaaaaatta attaattaga agatatatat atatatatat atatatacac atatatatgg 13740
   agtaattatt gagttgcacc ttgtacgaca ttatatcatt agcgaggcgt gcaatgatac 13800
   agacagctcg aacgattttg ggataggtag aggcccactc gagggcctcg ctagttgcca 13860
   catctcccat cgaaatgaac ccctggctca ccaagtgcat acagccactg ctgcgtgctg 13920
   aaattttcag atgttcatca acggtggctg gcacatattt cttgtcacgc cattctgtct 13980
   cggcattata gcacatggcc atgtcgatta tctaagatgc atgtgaaatt agtgtcaaat 14040
   aagctcaata actttataat ctaactatat actgttcgtt agcaaatgtc attaattttg 14100
   tcccgaacac aataacttct ttttataatc tactacacat gaaaagaaaa tttgttagaa 14160
   ctagattagt ttagatgttg cacacttgtt tgattagcat ataataggta aaaataaagc 14220
   agtgacaacc atattgcttt ttttgtccaa atttaaatgg ataacaatga atctagacac 14280
   gtatataata atacacatac atcaaatatt gcatgaattg attaacaccc taaaacgaat 14340
   actattttgg gacggagaag taccataata aaggttatag atgatgtcaa ccacaaggat 14400
   gtagaacgaa acatttgcac ataatgtaac cataaatgct tcacgacatt tacacataat 14460
   gtaaccataa ttattgattt atttctcagt tttaatcttg atgtttcaat tgagacatac 14520
   cagtcctttt accaactttg catgtttgtt gttttgaaaa ttcaagtcct cctcgatggc 14580
   ttttatggtg gcacgtatat ttgtatacag agtcctcaag tatgttgggc attgctccac 14640
   gccttcttca tcccacctgt catgtatgaa acttattatt gtattaccat gagtattaga 14700
   aaaatagaac ttttctagag agaggagctt tccatacaaa taaacaatta agactatata 14760
   aatatatcac tggaagcaag ggtaacagag aggattttag aatttcccat taatcaattt 14820
   taaatagtaa gagattctag cttcttcagg gctggtttgg tgacgggaga tttgaagggg 14880
   gtccctaggg aaagaaacct cttgagtttc ctcccctaga gatctccaaa tatatggtca 14940
   ccaaatcagt actcaattct ctccattaca cttgctccta aacaaagtca tttaagtcgc 15000
   atgatgaaaa tataaacata ctgtgaataa gtgttgcaaa cctttgtaat gctgcagtga 15060
   aggcgttgcc ttcctctgtg gtgcaatggc tgtcatacaa gtcatcgagt acagacacga 15120
   tcataaacaa ctgcgtgagc atctttcgtg aataggagta ttggggctca aagaagactc 15180
   ccatcatcca gaaatgcatc tccaccatcc tatctcgtgc atagattgtc gtgttagcct 15240
   gtgcttggaa ctctttccac cacctgttaa atacaatgta aatggtctta gtacaaaata 15300
   atgtggaaat gtgagtgaac taattaatgt gggactttga gagaacttag tactcccttc 15360
   tatctttttt tatttgtcac ggtttagtac aaaaataaac taacgagcga caaatattca 15420
   agaacggaag tagtacaaga ttggtatgct attaatgacg gttcttacag cgtaagatct 15480
   tttagctcct cacagtatag ggtgagcaag atgttgaagt ctaattttga gaactccagg 15540
   agcatattga tacgcgtagt gcttttctcg tatgttgaga tgaattgcct ggcctgtact 15600
   ctgttgagtc ttctgaaaag aggtgtctcc aatgcatatc taacttcttc ttgcagtgtt 15660
   ggttcgagat catctacaat acattgaagt tgtctcttgg tgtgaatgag aatattgtca 15720
   agtatttctt caccgttcac tctaacatgt gccgcttcgt acatcctcag taaacacctt 15780
   gcatcatttg ttacgatatc tcctttatca tctctgaact tcaaaaatac atctgcattc 15840
   gacactaaca acatgtgaga catccaccta tatatgaaac agttatacga ccgtagatgt 15900
   ggcggttata gaataatagg acagatccat tgttcaacct tcaacttgaa ccttagttcg 15960
   attttgaacc tttattattt taaataagat agaaaccacc tttgaaacaa gctaaagaga 16020
   agaccaaatt tgcccaaaac tttgacgatt ggagggtgtc tagtacctga ttttatagtt 16080
   cagggtttaa aattggaccg ttgtgcatgt atgttctgtc ctctttataa actaatgata 16140
   ctacaattag catttagcgt tatatatata tatatatata tatatatata tatatatata 16200
   tatatatata tatatatata tatcgccaca atatatttgt agtaaaaaaa cccctttgtt 16260
   gcttgggtgg aagatgtaga ccaaaaaaag tcctaatgtt atttgaagtt ccaactaata 16320
   natatacgcc aactataata gcaaaacatg tctgcaagct taccagaaga aaatatgata 16380
   tccatgcttc ctaagcaaat agaaaagctg tgatgttgta gtaagatcaa gatcttgatc 16440
   cctagcttcg aagacattgc acattaattc atcaatctcc ttggtatagt ggtaatccag 16500
   tccaagccgt tctagtgtat caatgcagtc taacttatga gacaaatcag aggatccaac 16560
   atccagtaac attttcttca cctcctcctt cattatttcg acgttatcct tcataatcaa 16620
   atactgcaag aaagatattt catctaattt ggttattctc ttattgtcta tagtgtaaac 16680
   ctttcatttg cacaacacag tacgagtcat atattttact tgaagagaca cctggttgtt 16740
   gatggaggcc agtgtgaatt aactagagaa accaatatga acaaattcat agcatagcgt 16800
   gaaatcagct agaaaaaacc ctctatatat gtcaactgcc ttataaatat gtgtagggtg 16860
   taaaagaaaa agaggtgcta gttgtgtgca tcacattaat tgggttgttt gattgattct 16920
   atatatctaa taacataaaa gtaatatcta tattggtatt acattatatt ttgaaaatag 16980
   ttggaaagtg gctagagaag atgacggagg gagagagaaa aagagagatc tagttgtttc 17040
   agaccttata ggaggatata atcaattttg attaagggtg ggccaaataa atatgatggc 17100
   attcatggat cttgtagatc gcacccgttc agtatttaag tttgaaattt ctctatatct 17160
   agaggcatgt ttggtatacc taaagtttag ttttgagact aaagtttagt tcgtacccta 17220
   tttggtttta ggaactaaaa gtattcagaa cgcattaaat tgactcataa gaagactaaa 17280
   atatctctta acattctcta gtcactagtg caactgaaat aaataagagg caacaagtga 17340
   aaataatatg ttttagttac actttaaggg actaaagact aaaacagttt agtcacaccc 17400
   gtttaaaaat ttaggaacta aatgagacta aaataaaatg gagggactag tgattaatcc 17460
   cttaaaccac acgggaccct aagaaatctc atctagatcc cccaattatt gcatgcttgt 17520
   ttagttctgt cagattaagg taattggatc cagctcgtac tgttcgaccc ttttgcatct 17580
   agtagtttcg ttcttttgct tacctactac cctacaaggt tcagataaaa aaatgtgtat 17640
   gggcgatcaa cattctgtcc atttgtttac caatgattac caaatcacat ccggatggcg 17700
   attactgtag tagcattttg caaacttggg acatgtttgg aacaactcct ccaatgaata 17760
   atctatatct tgaattcaat atatagcagc tccaatggtg gagctaagat ccacttggag 17820
   aactgtttgc caacacaaca gctttagatc aacatccacc taatggcatg aaactgactc 17880
   ttttgtcctt gttgggttgg ccttatgtca ttgtcgtctg tcatccattg ttcctcgtta 17940
   ggcaaaaaaa gtgacgcgcg tgggcgcaca ggcggacatg aacgtggcca ctggatcgag 18000
   aatgtggcca cagttggatg tggagaggca gagggaagaa gaagataaca gcattggagg 18060
   tgggcacaac tgaacgctgc ctcgttgggg gcgtggcctc gctggagccg tgtggaacat 18120
   ttatattctt gcatgtgtta tgtgagaagt ataggagtgc aggagattta gtcccacctt 18180
   actagttaag gtggagggaa cccaacttaa ataatcgagt actctctata ttcttgcatg 18240
   tgttgtatga aaagtataag gaaaaacata cgcgctcgct cccttcacct cgtcatcttt 18300
   aatctcgtca ccatggctcg tttgtgtcct cgtcacgtca ttgttggcaa caacgaaaca 18360
   aaacaagcgt acaacaacat tatgcatata tttagatctg ttttattttt tgattaaatt 18420
   aaaaccacat gttctatttt ttccaacata tatttgtcat gaaactactc atttcatgaa 18480
   acttggcatg gtatgtaata ggtgctttat cgtattgttt ggtctttcta cacatttccc 18540
   ttttttcaat ttttgagact attttttgtt catagggatt tcgacacctt ggctcacaaa 18600
   cagatgtggg aaacaagata acttagggtc cgtttggcag ggctccgcct tgcccaaaaa 18660
   tagctctacc tctggctttt cttgaaaacc aactatttta aagctgaagc tatttcacaa 18720
   attgtttggc agaaacgact tatctagagc cagagccatt tccctgtatt aaagtgctag 18780
   agccaaagcc aaaaccggtg aagttactat ttatggcttc ttctccgcgg tacaaaaatg 18840
   actctaactt ttaggtgctt ttcacattaa gtcgtttaaa aaaggtgttt ggcagggctc 18900
   tagcttttct ctagagaaaa gctagagccg atgccctacc aaacatgtac ttagataaga 18960
   ctcccggacg gaagtgctca tggggaaaca gatgtccaca aaaatccctc tcatagaatc 19020
   cactagcttt gaacctagcc cattatggtg gagggctacc aagtcaatca tcggattcat 19080
   cttacaccct ttcttgaaga tcgtttccac attagaagga cgtgtattct tcatctcatg 19140
   attaggttgt ctctagcgca tacggtaaac caccccttac gtagaggaag ctcatgatat 19200
   cctagggagg ggagggcagg agctggcgac gatgtcgcga gggaagggcc gcggtcggcg 19260
   acgggggagg ggtggctggg tcgcatggta gagggatggg gcagtgggat tgcgagggag 19320
   gggagatata tgagggatga aagatgatct aaataacatt gaccacataa tgtgagtgtg 19380
   taagatagag gatggtatcc aacaatctca actgttagtt ttgaaggtgt gatatgtgtc 19440
   tgtgcaattc gtttgatgga tttaggggag gttataggta tgaggtgatt tggttagatg 19500
   ggttttgcaa agttaaaact agtgggttat ataggcgtat agatagagtg taatgttttc 19560
   tcctctatat gttctagcgg tatcctacaa agtatcatct aaatataaag gatgttcctt 19620
   catcctctct atagagattt tctcttttct ccgctatcta ttttatactt tacacaacag 19680
   agtaattaag aaaactaaaa tatttgagag catgataaat atgtctataa tatatgtttt 19740
   taatcgtata ggaggagata tagagaacac caatataaag gaatggaata tagggcaaaa 19800
   aatcttttaa aagagtctat aaatgagaga gagaccgcga atactgagga tatggttgaa 19860
   tatagtctta tataactatt ttgctaagat attagagaca gaagtaataa ccgagcactc 19920
   tcttttattg caatataatg ataatttgga atcatctaac aaactcatac tactacatac 19980
   ttgtcgtgga gggcatggga catgatgaag gaagaagtca ccccagagtg gagtggtact 20040
   tgccatccat gtcctcactg tgtgagccta gcagggtagc gcagatatac atggcattgg 20100
   caggcacctc catcctacta tgttgttctt atagacagcg tggagtggta catatgatgc 20160
   aacatacgta cgcagttttg cattttaatt attgtttttt tttctggacg cagatatgca 20220
   agtatgcaac gtacacatac acgcagagct gccaacagac atgcaacacc tgtagctaaa 20280
   tccagtgcga gaactacatc aatacgtttc atgttttgtc atcacagtag ctttagatat 20340
   ccccacctaa tggcattttg attggaacta atacctcttt tgtccttggg tatatagtag 20400
   gcctcatgtc actgtctgcc gtctgtttct tcctcgttag acaaaaaaaa gtgacccagc 20460
   tgaagcatcg gggttttgga tgtgcatgtg gggttttgga tgcgccgaag gtggacgcga 20520
   cctcgctgtt gccaggtgcc taagaggcct aaagttagca cccttgtcac actttagggg 20580
   tgcctaagac tatttatagc agtctatcta aaatttatct aaaaatattg ttttatactg 20640
   ttctgtactg tagactatac tgtttgtaga gtagagttta attatggata ataatatggg 20700
   taaactgctg gagatagcct aaaggcctaa agcaaggata acttgcgtta ggcaatttgt 20760
   tctgttatgt tgtggcgtgt gttgttatgt ccaaatcgaa cgttttatta ggtgtggtgt 20820
   aattttgtat ggcaagctta aaaaatcttg ctaaactttt tacggccatc taagctagct 20880
   tactaaggaa ccaaacacta atctatggaa ccgcaatgtg gctaacatca gactatgtca 20940
   gttgggacac accctttcat ctgacagagt gcaggccata catatgtgcc attggtggcg 21000
   ttgacaccgt tggcagctcg tggctgttgt tggttcttgg tgaagatatt aacagctagc 21060
   acgtttggtt tgaggaatga agatcatttt ctcgctcctc gtattttctc gctcctcgtt 21120
   ttttgttttg gtttttggaa tggaataagt taatctatca ccatttcatt cctcatatgc 21180
   tagtgtaatt agtaataatg tgatgaatga gttcgttcca ctaaattagt agaatagact 21240
   catgagacag cacctcatct agaatatatt gatttttcaa accaaacact ccctaaatct 21300
   ttaggggtgg acattcttgc cttgaagtta aatgcatcgc agtgtagaag ctcaatcctt 21360
   ctagctacct tatggatttc tacaatgagg agtgtatcaa tgcttgtacc gtattattag 21420
   atgacatata tttctcatca aactttttcc ccctcaaatc ttttcatgat tattcgtaag 21480
   ctattaagaa atatatttaa ttattccaca ataatagaag acgtgctctc tcaaaacaca 21540
   agtgcatcga tgcaatagta ctagtgttga tagagacaat taaatatatt tcttggtctt 21600
   taaaccataa gtggttacgc actgtatatt aggacagaag gatactttat catattcttt 21660
   gttttttcaa ggcttttcca tatatttttt tttcaaattt tgatactagt tttgttcata 21720
   gggatttcga ccccttccca aacagatgcg agatctaagg ccgtgtttta ttcgcagcaa 21780
   atttttggaa tccggctatt tccattaccc gagactatct cgttcgacgc tgataatgga 21840
   gttctctaca cactagatat ccgagatgta aaatacacag tgtttgtttt atcgtccaga 21900
   ttctcatggc agattttttc aatatctcac cgaaacaaac acgtgctaaa atctaaagta 21960
   tattatgtcg tctataatat aagtaattaa cgtatagaga aggtgatata gagaactttg 22020
   tggagagaat tgagatatag aggatgaaat cttttagatg aatctataaa gatgagataa 22080
   tatggtcaga gacattcgta catataacta ttttgccaag atattagagg tagcaatagt 22140
   acgtaataac tgagcacact ctcttttatt tcaatataat ggtaatttgg gattatctca 22200
   agcaaaaaaa ctccagtaat gattaataac aaaagccgga gaggatggat ttgttcaaca 22260
   tactatacgt acctgtcctg gacgacatgg gacatggtga aggaagaagt caccccatag 22320
   agtggagtgg tgcttcccat gcatgtcctc ctcactgtgg agcctgctga cggatcttgc 22380
   ctcatcagct gccatgaaag tatagtatac tagctagcta ctctcctgct gcctgttgca 22440
   cgtacgtgcc caccggcgta gcagtgcaga tatacaatgc atgcactggc aagcacctgg 22500
   atcctatggt gttcttaata taggtagcca gcgtggagtg gtactcgtga tgcacgtggg 22560
   cacgtacgta ctcaatttgc attatattgt tttttttcct ggaaggatct gagcgtcgta 22620
   gtccactcgt ttttttttaa atatagatc 22649

<210> 10
<211> 21
<212> DNA
<213> artificial sequence

<220>
<223> primer oligonucleotide

<400> 10
gaacttcaaa aatacatcag a 21

<210> 11
<211> 25
<212> DNA
<213> Artificial sequence

<220>
<223> primer oligonucleotide

<400> 11
gacggatctt gcctcatcag ctgcc 25

<210> 12
<211> 25
<212> DNA
<213> Artificial sequence

<220>
<223> primer oligonucleotide

<400> 12
gtatactagc tagctactct cctgc 25